# EUROPEAN PATENT APPLICATION

(11) **EP 4 711 364 A1**
(43) Date of publication of application: **18.03.2026**
(21) Application number: 24806458.6
(22) Date of filing: 10.05.2024
(51) Int. Cl.: C07D 471/04, C07D 487/00, C07D 519/00, A61K 31/395, A61P 35/00

(54) **FUSED PYRROLE RING OR FUSED PYRIDINE RING COMPOUND**

(30) Priority: 12.05.2023 CN 202310534513
(71) Applicant: Pharmaengine, Inc., Taiwan 10480 (TW)
(72) Inventor: LIU, Cheng-Hao, Taipei 104 Taiwan (TW); LIN, Long-Zhi, Taipei 104 Taiwan (TW); WANG, Hong-Ren, TAIPEI 104 Taiwan (TW); XUN, Guoliang, Shanghai 201315 (CN); SUN, Yina, Shanghai 201315 (CN); YANG, Jinyu, Shanghai 201315 (CN); ZHOU, Jianlai, Shanghai 201315 (CN); CHEN, Bin, Shanghai 201315 (CN)
(74) Representative: Engelhard, Markus
(86) International application number: PCT/CN2024/092155
(87) International publication number: WO 2024/235106

(57) **Abstract**

The present disclosure provides a compound of formula (I): 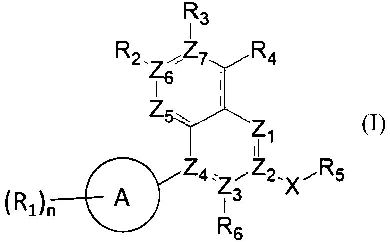 wherein ring A, Z₁, Z₂, Z₃, Z₄, Z₅, Z₆, Z₇, X, R₁, R₂, R₃, R₄, R₅, R₆, and n are as disclosed in the specification. The compound of formula (I) is for use as a Myt1 inhibitor. The present disclosure also provides a pharmaceutical composition comprising the compound of formula (I) and a method of treating cancer using the compound. The present disclosure further provides a process for preparing the compound of formula (I).

## Description

### FIELD OF THE APPLICATION

The present disclosure relates to a method for using inhibitors of membrane-associated tyrosine- and threonine-specific cdc2-inhibitory kinase (Mytl) (gene name PKMYT1), e.g., for the treatment of diseases or conditions, e.g., cancers, and, in particular, diseases or conditions that are dependent on the activity of Mytl (e.g., cancers with amplification/over-expression of CCNE1 or cancers with FBXW7 mutations). Specifically, the present disclosure relates to compounds comprising fused pyrrole rings or fused pyridine rings that function as Mytl inhibitors, a pharmaceutical composition comprising the compounds and a method of using the same.

### BACKGROUND

DNA is constantly subjected to internal (e.g., stalled replication forks and reactive oxygen species) or external (ultraviolet ray, ionizing radiation and chemicals) damage that may lead to DNA damage. In response to these potential dangers, cells have evolved complex mechanisms to counteract these deleterious events in order to prevent them from compromising genome integrity and thereby causing diseases associated with genome instability, such as cancers. These mechanisms are collectively known as DNA damage response (DDR).

DDR regulates specific DNA repair mechanisms at various stages of the cell cycle by activating various checkpoint pathways, including G1, S, G2, and mitotic checkpoints. Due to p53 mutation, most cancer cells have lost the G1 checkpoint, so it is necessary to rely on the G2 checkpoint for DNA damage correction before entering mitosis and dividing into two daughter cells.

MYT1 (myelin transcription factor 1) inhibits the cell cycle process by inhibiting the activity of cell cycle-related proteins such as cyclin A, CDKl (i.e., cdc2) and CDK2, allowing various tumor cells to repair their DNA damage and continue to proliferate.

Cdc2 (i.e., CDK1) is a cyclin-dependent kinase that controls the entry of cells into mitosis. During the G2-M transition, cdc2 is dephosphorylated at Tyr-15 and Thr-14, allowing mitosis to proceed. Since phosphorylation of cdc2 on threonine 14 (Thr-14) and tyrosine 15 (Tyr-15) inhibits the activity of the enzyme and prevents premature initiation of mitosis, the timing of cdc2 dephosphorylation is essential for the initiation of mitosis.

By arresting the cell cycle at the G2 phase, cells can repair DNA damage before mitosis to avoid death. Studies have shown that if cdc2 is dephosphorylated prematurely, it will lead to mitotic catastrophe and cell death. Mytl is an important cell cycle regulator in the G2-M phase. By inhibiting Mytl to block the phosphorylation of cdc2 at Tyr-15 and Thr-14, cdc2 can prematurely initiate cell mitosis and lead to the death of rapidly proliferating cells. Moreover, inhibiting Mytl can prevent tumor cells from repairing DNA and producing resistance to chemotherapy drugs. In summary, inhibiting Myt1 can reduce tumor cell proliferation and has therapeutic benefits. It can also overcome the resistance of tumor cells to chemotherapy drugs by combining Mytl inhibitors with chemotherapy drugs.

Therefore, there is a need to develop a potent Mytl kinase inhibitor for the treatment of cancers.

### SUMMARY OF THE INVENTION

One aspect of the present disclosure is to provide a compound of formula (I): 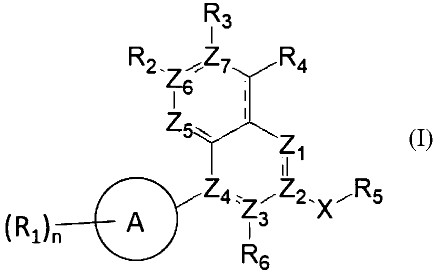
wherein ring A, Z₁, Z₂, Z₃, Z₄, Z₅, Z₆, Z₇, X, R₁, R₂, R₃, R₄, R₅, R₆, and n are as disclosed in the specification,
or a pharmaceutically acceptable salt, geometric isomer, enantiomer, diastereomer, racemate, prodrug, solvate, or hydrate thereof.

Another aspect of the present disclosure is to provide a compound of formula (IC): 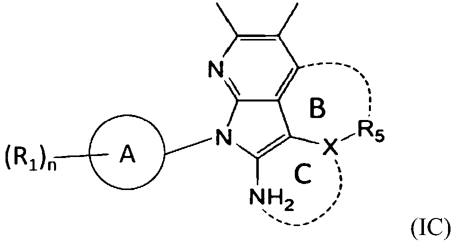
wherein R₁, n, ring A, ring B, ring C, X, and R₅ are as disclosed in the specification,
or a pharmaceutically acceptable salt, geometric isomer, enantiomer, diastereomer, racemate, prodrug, solvate, or hydrate thereof.

A further aspect of the present disclosure is to provide a compound of formula (II): 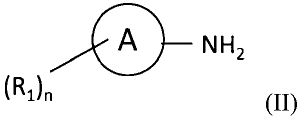 wherein ring A, R₁ and n are as disclosed in the specification.

The compound of formula (II) is used as a starting compound for preparing the compound of formula (I).

Another aspect of the present disclosure is to provide a pharmaceutical composition, which includes a therapeutically effective amount of a compound of formula (I) or a pharmaceutically acceptable salt, geometric isomer, enantiomer, diastereomer, racemate, prodrug, solvate, or hydrate thereof, and one or more pharmaceutically acceptable carriers or excipients thereof. The pharmaceutical composition of the present disclosure is used as a Mytl inhibitor.

Another aspect of the present disclosure is to provide use of a compound of formula (I) or a pharmaceutically acceptable salt, geometric isomer, enantiomer, diastereomer, racemate, prodrug, solvate, or hydrate thereof in the manufacture of a medicament used as a Mytl inhibitor.

Another aspect of the present disclosure is to provide a method for treating a cancer, which includes administering to an individual in need thereof a therapeutically effective amount of a compound of formula (I) or a pharmaceutically acceptable salt, geometric isomer, enantiomer, diastereomer, racemate, prodrug, solvate, or hydrate thereof, to inhibit growth of the cancer in the individual. The compound of formula (I) or a pharmaceutically acceptable salt, geometric isomer, enantiomer, diastereomer, racemate, prodrug, solvate, or hydrate thereof may be used alone or in combination with another therapeutic agent and/or therapy.

Another aspect of the present disclosure is to provide the use of a compound of formula (I) in the manufacture of a medicament for treating cancer. In one embodiment, the medicament further comprises another therapeutic agent, or the medicament is used in combination with another therapeutic agent and/or therapy.

Another aspect of the present disclosure is to provide a compound of formula (I) for use in treating cancer.

Another aspect of the present disclosure is to provide a pharmaceutical composition for treating cancer, comprising a compound of formula (I).

Still, another aspect of the present disclosure is to provide a method for preparing a compound of formula (I) or a pharmaceutically acceptable salt, geometric isomer, enantiomer, diastereomer, racemate, prodrug, solvate, or hydrate thereof.

### DETAILED DESCRIPTION

The present disclosure may be more readily understood by reference to the following detailed description of various embodiments, examples, and tables having their relevant descriptions of the present disclosure. Unless otherwise defined, all terms (including technical and scientific terms) as used herein have the same meaning as is commonly understood by those of ordinary skill in the art to which the present disclosure belongs. Furthermore, it is to be understood that terms such as those defined in commonly used dictionaries are to be construed to be consistent with their meanings in the context of relevant fields, and that said terms are not to be construed in an idealized or overly formalized sense unless expressly defined herein. It should also be understood that the terms as used herein are for the purpose of describing particular embodiments only and are not intended for limitation.

### Definition

The definitions set forth in this section are intended to clarify terms used throughout this application. The term "herein" means the entire application.

It is important to note that singular forms "a/an" and "the", as used herein, include plural references, unless otherwise expressly provided herein. Accordingly, unless otherwise required herein, singular terms shall include plural forms, and plural terms shall include singular forms.

Typically, a range herein is expressed as from "about" one particular value and/or to "about" another particular value. When expressing such ranges, one embodiment includes a range from one particular value and/or to another particular value. Similarly, when values are expressed as approximations through the use of the word "about", it should be understood that the particular value constitutes another embodiment. Furthermore, it should be understood that an endpoint of each of the ranges is meaningful both relative to and independent of the other endpoint. As used herein, the term "about" means +20%, preferably +10%, and even more preferably +5%.

As used herein, the term "optionally substituted" means that the substitution is optional. In case that the substitution is desired, such substitution means replacement of any number of hydrogens on a designated atom by an option from a designated group, provided that the normal valence of the designated atom is not exceeded and the substitution produces a stable compound. For example, when a substituent is a ketone group (i.e., =O), 2 hydrogens on the atom are replaced. Examples of substituents of a "substituted" group are those present in exemplary compounds and embodiments as disclosed herein and may include, for example, halogen, cyano, alkyl, alkoxy, haloalkyl, alkylamino, aminoalkyl, dialkylamino, hydroxyalkyl, alkoxyalkyl, hydroxyalkoxy, alkoxyalkoxy, aminoalkoxy, alkylaminoalkoxy, alkylaminoalkyl, and the like.

As used herein, the term "halogen" includes fluorine, chlorine, bromine and iodine. The term "halo" used as a prefix to a group means that one or more hydrogens on the group are replaced by one or more halogens.

As used herein, the term "alkyl" means a monovalent, saturated, straight or branched hydrocarbyl containing 1 to 12 carbon atoms. Preferably, the alkyl is C₁-C₈ alkyl. More preferably, the alkyl is C₁-C₆ alkyl. The alkyl may be unsubstituted or substituted with one or more substituents. Examples of C₁-C₆ alkyl include, but are not limited to: methyl, ethyl, n-propyl, isopropyl, n-butyl, isobutyl, sec-butyl, tert-butyl, pentyl (including all isomeric forms), and hexyl (including all isomeric forms), heptyl (including all isomeric forms), octyl (including all isomeric forms), and the like.

The term "alkoxy", used alone or as a suffix or prefix, means a group of the general formula -O-(alkyl), where the alkyl is defined above. Exemplary alkoxy includes, but is not limited to: methoxy, ethoxy, n-propoxy, isopropoxy, n-butoxy, isobutoxy, sec-butoxy, tert-butoxy, and the like.

As used herein, the term "alkenyl" means a non-cyclic monovalent straight or branched hydrocarbyl containing one, two or three carbon-carbon double bonds. Non-limiting examples of alkenyl include vinyl, prop-1-enyl, prop-2-enyl, 1-methylvinyl, but-1-enyl, but-2-enyl, but-3-enyl, 1-methylprop-1-enyl, 2-methylprop-1-enyl and 1-methylprop-2-enyl. Alkenyl may be optionally substituted as defined herein for alkyl.

The term "amino" used as a prefix or suffix to a group means that one or more hydrogens on the group are replaced by one or more aminos.

As used herein, the term "alkylamino" means a group having the formula -N(RN₁)₂ or - NHRN₁, where RN₁ is the alkyl as defined herein. An alkyl moiety of the alkylamino may be optionally substituted as defined for alkyl. Each of the optional substituents on the substituted alkylamino itself may be unsubstituted or, if the valence permits, substituted by an unsubstituted substituent as defined herein for each corresponding group.

Unless the number of rings is otherwise indicated, the term "cycloalkyl" as used herein means a saturated monovalent hydrocarbyl having a cyclic configuration, including monocyclic, bicyclic, tricyclic, and higher polycyclic alkyls (and, in the case of polycycle, including fused and bridged bicyclic and spirocyclic moieties), where each of the cyclic moieties has 3 to 12 carbon atoms. Preferably, the cycloalkyl has 3 to 8 carbon atoms. More preferably, the cycloalkyl has 3 to 6 carbon atoms. When the cycloalkyl contains more than one ring, the rings may be fused or unfused and include a bicyclic group. A fused ring usually means at least two rings that share two atoms therebetween. Such cycloalkyls include, for example, a monocyclic structure such as cyclopropyl, cyclobutyl, cyclopentyl, cyclohexyl, cycloheptyl, cyclooctyl, 1-methylcyclopropyl, 2-methylcyclopentyl, 2-methylcyclooctyl, or a bicyclic, polycyclic or bridged cyclic structure, such as adamantyl.

Unless the number of rings is otherwise indicated, the term "aryl" means a 6- to 14-membered monocyclic or bicyclic carbon ring, where the monocyclic ring is an aromatic ring and at least one of the rings in the bicyclic ring is an aromatic ring. Representative examples include, but are not limited to: phenyl, biphenyl, naphthyl, indenyl, and the like.

Unless the number of rings is otherwise indicated, "heteroaryl" means a monovalent group having 5 to 14 ring atoms of a monocyclic, fused bicyclic or fused tricyclic ring including one or more, for example, one, two, three or four, ring heteroatoms independently selected from: -O-, -S(O)ₙ- (n is 0, 1 or 2), -N-, -N(R^{x})-, and the remaining ring atoms being carbon, where the ring including a monocyclic group is an aromatic ring and where at least one of the fused rings including a bicyclic or tricyclic group is an aromatic ring. One or two ring carbon atoms of any non-aromatic ring including a bicyclic or tricyclic group may be replaced by a -C(O)-, -C(S)- or -C(=NH)- group. R^{x} is hydrogen, alkyl, hydroxyl, alkoxy, acyl, or alkylsulfonyl. The fused bicyclic group includes a bridged ring system. Unless otherwise stated, the atomic valence may be located on any atom of any ring of the heteroaryl as long as the atomic valence rule permits. Specifically, R^{x} does not exist when the atomic valence is located on nitrogen. More specifically, the term "heteroaryl" includes, but is not limited to: benzocyclopentyl, 2,3-dihydrobenzofuranyl, 2,3-dihydrofuro[3,2-b]pyridine, 2,3-dihydrofuro[3,2-c]pyridine, 6,7-dihydro-5H-cyclopenta[b]pyridine, chromanyl, isochromanyl, 3,4-dihydro-2H-pyrano[2,3-b]pyridine, 5,8-dihydro-6H-pyrano[3,4-b]pyridine, 1,3,4,5-tetrahydrobenzo[c]oxepine, indanyl, phthalidyl, indolinyl, 1,2,4-triazolyl, oxo-1,3,5-triazolyl, phthalimido, pyridyl, pyrrolyl, imidazolyl, thiophenyl, furanyl, indolyl, 2,3-dihydro-1H-indolyl (including, for example, 2,3-dihydro-1H-indol-2-yl or 2,3-dihydro-1H-indol-5-yl, and the like), isoindolyl, dihydroindolyl, isoindolinyl, benzimidazolyl, benzodioxol-4-yl, benzofuranyl, cinnolinyl, indolizinyl, naphthyridin-3-yl, phthalizin-3-yl, phthalizin-4-yl, pteridinyl, purinyl, quinazolinyl, quinoxalinyl, tetrazolyl, pyrazolyl, pyrazinyl, pyrimidinyl, pyridazinyl, oxazolyl, isoxazolyl, oxadiazolyl, benzoxazolyl, quinolinyl, isoquinolinyl, tetrahydroisoquinolinyl (including, for example, tetrahydroisoquinolin-4-yl or tetrahydroisoquinolin-6-yl, and the like), pyrrolo[3,2-c]pyridinyl (including, for example, pyrrolo[3,2-c]pyridin-2-yl or pyrrolo[3,2-c]pyridin-7-yl, and the like), benzopyranyl, thiazolyl, isothiazolyl, thiadiazolyl, benzothiazolyl, benzothienyl, and derivatives thereof, or an N-oxide, or protected derivatives thereof.

Unless the number of rings is otherwise indicated, "heterocyclic" means a saturated or partially unsaturated monovalent monocyclic group having 3 to 9 ring atoms or a saturated or partially unsaturated monovalent fused bicyclic group having 5 to 12 ring atoms, where one or more, for example, one, two, three or four, ring heteroatoms are independently selected from: - O-, -S(O)ₓ- (x is 0, 1, or 2), -N=, -N(R^{y})- (where R^{y} is hydrogen, alkyl, hydroxyl, alkoxy, acyl or alkylsulfonyl), and the remaining ring atoms are carbon. One or two ring carbon atoms may be replaced by a -C(O)-, -C(S)- or -C(=NH)- group. As used herein, the term "heterocyclyl" means a 3-, 4-, 5-, 6-, 7- or 8-membered monocyclic, bicyclic, tricyclic or tetracyclic ring system having a fused, bridged and/or spirocyclic ring which contains, unless otherwise indicated, one, two, three or four heteroatoms independently selected from nitrogen, oxygen and sulfur. The fused bicyclic group includes a bridged ring system. Unless otherwise stated, the atomic valence of a group may be located on any atom of any ring within the group as long as the atomic valence rule permits. Specifically, R^{y} does not exist when the atomic valence is located on a nitrogen atom. More specifically, the term "heterocyclyl" includes, but is not limited to: piperidinyl, pyrimidinyl, morpholinyl, piperazinyl, acridinyl, pyrrolidinyl, 2-oxopyrrolidinyl, 2,5-dihydro-1H-pyrrolyl, 4-piperidinonyl, 2-oxopiperazinyl, tetrahydropyranyl, 2-oxopiperidinyl, thiomorpholinyl, thiomorpholinyl, perhydroazepinyl, pyrazolidinyl, dihydroimidazolyl, imidazolidinyl, dihydropyridyl, tetrahydropyridyl, oxazolinyl, oxazolidinyl, isooxazolidinyl, thiazolinyl, thiazolidinyl, quinuclidinyl, isothiazolidinyl, octahydroindolyl, octahydroisoindolyl, decahydroisoquinolyl, tetrahydrofuryl and tetrahydropyranyl, and derivatives thereof, and an N-oxide or protected derivatives thereof.

As used herein, the term "pharmaceutically acceptable salt" means a derivative of a disclosed compound, where the parent compound is modified by preparing a pharmaceutically acceptable acidic salt or basic salt thereof. Examples of pharmaceutically acceptable salt include, but are not limited to: mineral salts or organic acid salts of basic residues (e.g., amines); alkali metal salts or organic salts of acidic residues (e.g., carboxylic acids); and the like. Pharmaceutically acceptable salts include conventional non-toxic salts or quaternary ammonium salts formed from the parent compound with, for example, non-toxic inorganic acids or organic acids. Suitable non-toxic acids include, but are not limited to, inorganic and organic acids such as acetic acid, alginic acid, anthranilic acid, benzenesulfonic acid, benzoic acid, camphorsulfonic acid, citric acid, ethanesulfonic acid, formic acid, fumaric acid, furoic acid, galacturonic acid, gluconic acid, glucuronic acid, glutamic acid, glycolic acid, hydrobromic acid, hydrochloric acid, isethionic acid, lactic acid, maleic acid, malic acid, amygdalic acid, methanesulfonic acid, mucic acid, nitric acid, pamoic acid, pantothenic acid, phenylacetic acid, phosphoric acid, propionic acid, salicylic acid, stearic acid, succinic acid, sulfanilic acid, sulfuric acid, tartaric acid and p-toluenesulfonic acid. Non-limiting examples of salts of the compound of the present disclosure include, but are not limited to: hydrochloride, hydrobromide, hydroiodide, sulfate, bisulfate, 2-hydroxyethanesulfonate, phosphate, hydrophosphate, acetate, adipate, alginate, aspartate, benzoate, butyrate, camphorate, camphorsulfonate, citrate, digluconate, glycerophosphate, hemisulfate, heptanate, hexanoate, formate, succinate, malonate, fumarate, maleate, methanesulfonate, mesitylenesulfonate, naphthalenesulfonate, nicotinate, oxalate, pamoate, pectinate, persulfate, 3-phenylpropionate, picrate, trimethylacetate, propionate, trichloroacetate, trifluoroacetate, glutamate, bicarbonate, undecanoate, lactate, citrate, tartrate, gluconate, benzenesulfonate and p-toluenesulfonate.

As used herein, the term "geometric isomer" includes, but is not limited to: cis- and transforms; E- and Z-forms; c-, t- and r-forms; endo- and exo-forms; R-, S- and mesomeric-forms; boat, chair, twisted, encapsulated and semi-chair forms; and a combination thereof.

As used herein, the term "enantiomer" means a pair of stereoisomers that are nonoverlapping mirror images of each other. A 1:1 mixture of a pair of enantiomers is a racemic mixture. The term "enantiomer" means, where appropriate, the racemic mixture. "Diastereomer" is a stereoisomer having at least two asymmetric atoms that are not mirror images of each other. Absolute stereochemistry can be designated according to the Cahn-Ingold-PrelogR-S system. When the compound is a pure enantiomer, the stereochemistry at each chiral carbon can be designated by R or S. Split compounds can be denoted as (+) or (-) depending on the direction (dextrorotatory or levorotatory) in which they rotate the plane polarization at the wavelength of the sodium D line. Certain compounds as described herein may contain one or more asymmetric centers or axes, and may thereby produce enantiomers, diastereomers and other stereoisomeric forms, which may be defined, in the case of absolute stereochemistry, as (R)- or (S)-. The present disclosure is intended to encompass all such possible isomers, including racemic mixtures, optically pure forms and intermediate mixtures. Optically active (R)- and (S)-isomers can be prepared using chiral synthetic building blocks or chiral reagents, or split using conventional techniques. If the compound contains a double bond, the substituent may be in the E or Z configuration. If the compound contains a di-substituted cycloalkyl, the substituent of cycloalkyl may have a cis or trans configuration.

As used herein, "prodrug" is intended to include any covalently bonded carrier that releases the active parent drug according to formula I via in vivo physiological action (e.g., hydrolysis, metabolism, and the like) when such prodrug is administered to an individual. The applicability and techniques involved in the preparation and use of the prodrug are well known to those of ordinary skill in the art to which they belong. A prodrug of the compound of formula (I) (the parent compound) may be prepared by modifying functional groups present in the compound in a manner that allows a variant to be cleaved to the parent compound in conventional operations or in vivo. "Prodrug" includes the compound of formula (I) in which a hydroxyl, amino or sulfhydryl is bonded to any group which, upon administration of the prodrug to the individual, cleaves to form a free hydroxyl, free amino or free sulfhydryl, respectively. Examples of prodrugs include, but are not limited to, derivatives and metabolites of the compound of formula (I), which include a biohydrolyzable portion, such as biohydrolyzable amides, biohydrolyzable esters, biohydrolyzable carbamates, biohydrolyzable carbonates, biohydrolyzable ureides, and biohydrolyzable phosphate analogs. In particular embodiments, the prodrug of the compound of formula (I) having a carboxyl functional group is a low-carbon alkyl (e.g., C₁-C₆) ester of carboxylic acid. Carboxylate is preferably formed by esterifying any of the carboxylic acid moieties present on the molecule.

As used herein, the term "solvate" means a compound or a pharmaceutically acceptable salt thereof further including stoichiometrically or non-stoichiometrically solvent bound by non-covalent intermolecular forces. If the solvent is water, the solvate is appropriately known as a "hydrate", e.g., hemihydrate, monohydrate, sesquihydrate, dihydrate, trihydrate, and the like.

As used herein, the term "Myt1 inhibitor" means a compound that reduces the activity of Mytl upon exposure to enzyme Mytl (whether in vitro, in cell cultures, or in animals), such that the measured Mytl IC₅₀ is 10 mM or less (e.g., 5 mM or less or 1 mM or less).

For some Mytl inhibitors, the Mytl IC₅₀ may be 100 nM or less (e.g., 10 nM or less, or 3 nM or less), and may be as low as 100 pM or 10 pM. Preferably, the Mytl IC₅₀ is 1 nM to 1 mM (e.g., 1 nM to 750 nM, 1 nM to 500 nM, or 1 nM to 250 nM).

Even more preferably, the Mytl IC₅₀ is less than 20 nm (e.g., 1 nM to 20 nM).

As used herein, "treatment" means the medical management of a subject for the purpose of ameliorating, improving, stabilizing, preventing or curing a disease or condition.

The term includes active treatment (treatment aimed to improve a disease or condition); causal treatment (treatment directed at the cause of a related disease or condition); palliative treatment (treatment aimed to alleviate the symptoms of a disease or condition); prophylactic treatment (treatment aimed to minimize or partially or completely inhibit the progression of a related disease or condition); and supportive treatment (treatment used for complementing another treatment).

### Compound

The present disclosure provides a compound of formula (I): 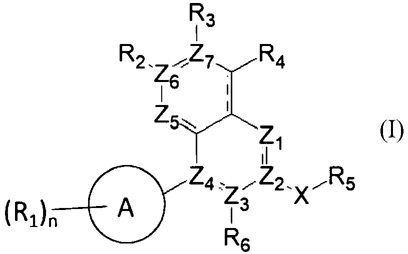 wherein:
ring A is indazolyl;
R₁ is halogen, hydroxyl, oxo, cyano, nitro, amino, alkyl, alkoxy, cycloalkyl, haloalkyl, haloalkoxy, halocycloalkyl, -B(OH)₂, -S(=O)-alkyl, -S(=O)₂-alkyl, -S(=O)NH₂, -S(=O)₂NH₂, - P(=O)-dialkyl, -C(=O)NH₂, -C(=O)alkyl, -C(=O)alkoxy, boronic acid group, heterocyclyl, aryl, or heteroaryl, wherein the alkyl, alkoxy, cycloalkyl, haloalkyl, haloalkoxy, halocycloalkyl, heterocyclyl, aryl, or heteroaryl groups are optionally substituted with substituents independently selected from halogen, hydroxyl, amino, alkyl, aryl, heteroaryl, or heterocyclyl;
Z₁ is a direct bond;
Z₂, Z₃, and Z₄ are independently CRₐ or N;
Z₅ is -C(=O)-, N, or CRₐ, wherein Rₐ is hydrogen, alkyl, hydroxyalkyl, alkenyl, alkynyl, amino, or cyano;
Z₆ and Z₇ are independently CRₐ or N;
wherein the position of the double bonds on the bicyclic ring connected to ring A varies depending on whether Z₂ to Z₇ are N or -C(=O)-; and, wherein Rₐ is hydrogen, alkyl, hydroxyalkyl, alkenyl, alkynyl, amino, or cyano;
X is -C(=O)- or NR_{c}, wherein R_{c} is heterocyclyl, aryl, or heteroaryl, wherein the heterocyclyl, aryl, or heteroaryl groups are optionally substituted with substituents independently selected from hydroxyl, amino, alkyl, aryl, heteroaryl, or heterocyclyl;
R₂, R₃, and R₄ are independently hydrogen, alkyl, -C(=O)NH₂, amino, alkylamino, or dialkylamino, wherein the alkyl, amino, alkylamino, or dialkylamino groups are optionally substituted with substituents independently selected from hydroxyl, amino, alkyl, aryl, heteroaryl, or heterocyclyl;
R₅ is alkyl, amino, alkylamino, or dialkylamino, wherein the alkyl, amino, alkylamino, or dialkylamino groups are optionally substituted with substituents independently selected from hydroxyl, amino, alkyl, aryl, heteroaryl, or heterocyclyl; and R₅ together with R₄ forms a 5- to 8-membered heteroaryl or heterocyclic ring, wherein the heteroaryl or heterocyclic ring is optionally substituted with substituents independently selected from halogen, cyano, alkoxy, carbonyl, hydroxyl, amino, alkyl, aryl, heteroaryl, heterocyclyl, or cycloalkyl groups;
R₆ is hydrogen, amino, alkylamino, or dialkylamino; or, R₆ together with the attached Z₃ forms a -C(=O)- group; and
n is 3 or 4;
or a pharmaceutically acceptable salt, geometric isomer, enantiomer, diastereomer, racemate, prodrug, solvate, or hydrate thereof.

The present disclosure further provides a compound of formula (IC): 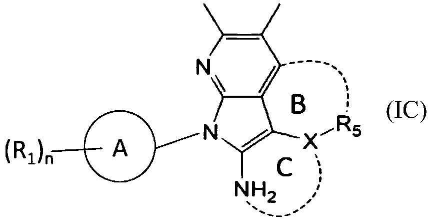 wherein:
ring A is indazolyl;
each R₁ is independently halogen, hydroxyl, oxo, cyano, nitro, amino, alkyl, alkoxy, cycloalkyl, haloalkyl, haloalkoxy, halocycloalkyl, -B(OH)₂, -S(=O)-alkyl, -S(=O)₂-alkyl, - S(=O)NH₂, -S(=O)₂NH₂, -P(=O)-dialkyl, -C(=O)NH₂, -C(=O)alkyl, -C(=O)alkoxy, boronic acid group, heterocyclyl, aryl, or heteroaryl, wherein the alkyl, alkoxy, cycloalkyl, haloalkyl, haloalkoxy, halocycloalkyl, heterocyclyl, aryl, or heteroaryl groups are optionally substituted with substituents independently selected from halogen, hydroxyl, amino, alkyl, aryl, heteroaryl, or heterocyclyl;
n is 3 or 4;
X is -C(=O)- or NR_{c}, wherein R_{c} is heterocyclyl, aryl, or heteroaryl;
R₅ is alkyl, amino, alkylamino, or dialkylamino, wherein the alkyl, amino, alkylamino, or dialkylamino groups are optionally substituted with substituents independently selected from hydroxyl, amino, alkyl, aryl, heteroaryl, or heterocyclyl;
ring B and Ring C do not exist simultaneously;
ring B is a 5- to 8-membered heteroaryl or heterocyclyl, wherein the heteroaryl and heterocyclyl are optionally substituted with substituents independently selected from halogen, cyano, alkoxy, carbonyl, hydroxyl, amino, alkyl, aryl, heteroaryl, heterocyclyl, or cycloalkyl; and
ring C is a 5- to 8-membered heteroaryl or heterocyclyl, wherein the heteroaryl and heterocyclyl are optionally substituted with substituents independently selected from halogen, cyano, alkoxy, carbonyl, hydroxyl, amino, alkyl, aryl, heteroaryl, heterocyclyl, or cycloalkyl;
or a pharmaceutically acceptable salt, geometric isomer, enantiomer, diastereomer, racemate, prodrug, solvate, or hydrate thereof.

In one embodiment, the compound of formula (I) is a compound of formula (IC).

In one embodiment of the compound of formula (I) or (IC), X is -C(=O)-.

In another embodiment of the compound of formula (I) or (IC), X is NR_{c}, wherein R_{c} is heterocyclyl, aryl, or heteroaryl.

In one embodiment of the compound of formula (I) or (IC), R₁ is selected from a group consisting of

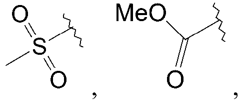

-CH₃,

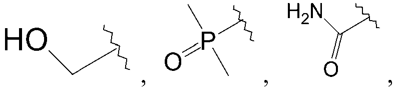

-CN,-OH,

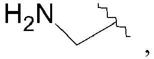

-OCH₃, -F, -Cl, -Br,

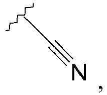

-CF₃, -CCl₃, -NO₂,

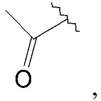

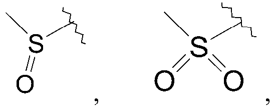

-B(OH)₂,

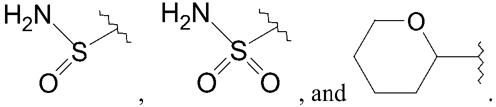

In one embodiment of the compound of formula (I) or (IC), Z₁ is a direct bond; Z₃ is C; at least one of Z₂ and Z₄ is N; Z₅ is -C(=O)-, N, or CRₐ, wherein Rₐ is hydrogen, alkyl, hydroxyalkyl, alkenyl, alkynyl, amino, or cyano; and Z₆ and Z₇ are independently C or N.

In one embodiment of the compound of formula (I) or (IC), R₂, R₃, and R₄ are independently hydrogen, alkyl, -C(=O)NH₂, or amino; R₅ is alkyl or amino; and R₆ is hydrogen or amino.

In one embodiment of the compound of formula (IC), R₅ is alkyl or amino.

In one embodiment of the compound of formula (I), ring A is indazolyl; each R₁ is independently hydrogen, halogen, hydroxyl, cyano, nitro, amino, unsubstituted alkyl, alkyl substituted with hydroxyl, alkoxy, alkylamino, haloalkyl, -S(=O)-alkyl, -S(=O)₂-alkyl, - S(=O)NH₂, -S(=O)₂NH₂, -P(=O)-dialkyl, -C(=O)NH₂, -C(=O)alkyl, -C(=O)alkoxy, or boronic acid group; and n is 3 or 4.

In one embodiment of the compound of formula (IC), X is -C(=O)-; R₅ is alkyl or amino; ring B is a 6- or 7-membered heteroaryl or heterocyclyl, wherein the heteroaryl and heterocyclyl groups are optionally substituted with substituents independently selected from halogen, cyano, alkoxy, carbonyl, hydroxyl, amino, alkyl, aryl, heteroaryl, heterocyclyl, or cycloalkyl; and ring C does not exist.

In a further embodiment of the compound of formula (I) or (IC), ring A is indazolyl; X is -C(=O)-; and R₁ is selected from a group consisting of

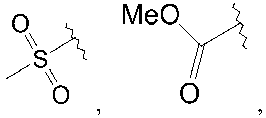

-CH₃,

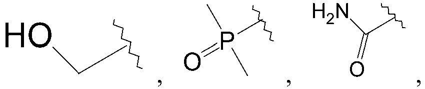

-CN, -OH,

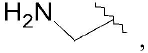

-OCH₃, -F, -Cl, -Br,

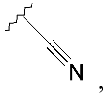

-CF₃, -CCl₃, -NO₂,

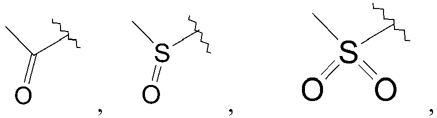

-B(OH)₂,

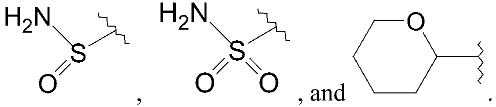

In another preferred embodiment, the present disclosure provides a compound of formula (I) selected from:

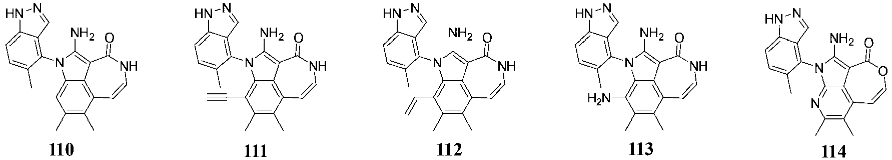

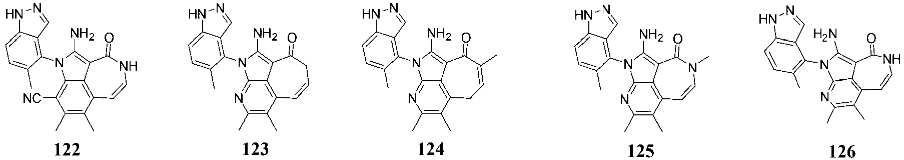

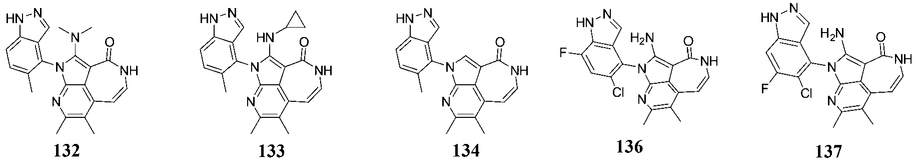

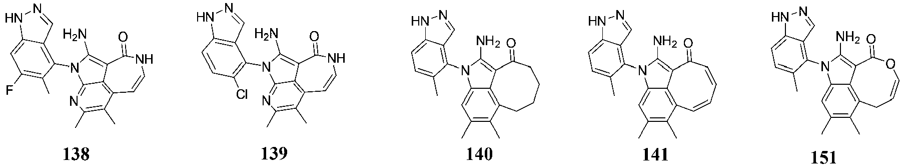

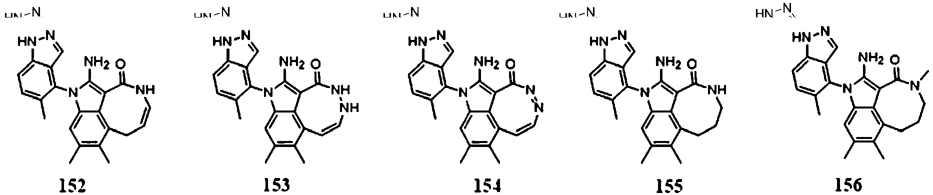

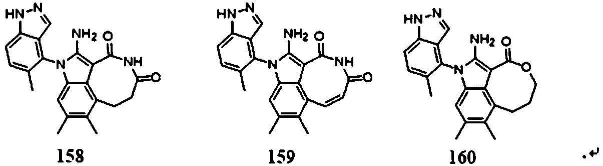

The present disclosure also provides a compound of formula (II): 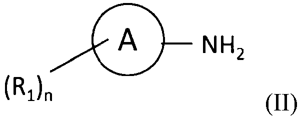 wherein:
ring A is indazolyl;
R₁ is halogen, hydroxyl, oxo, cyano, nitro, amino, alkyl, alkoxy, cycloalkyl, haloalkyl, haloalkoxy, halocycloalkyl, -S(=O)-alkyl, -S(=O)₂-alkyl, -S(=O)NH₂, -S(=O)₂NH₂, -P(=O)-dialkyl, -C(=O)NH₂, -C(=O)alkyl, -C(=O)alkoxy, boronic acid group, heterocyclyl, aryl, or heteroaryl, wherein the alkyl, alkoxy, cycloalkyl, haloalkyl, haloalkoxy, halocycloalkyl, heterocyclyl, aryl, or heteroaryl groups are optionally substituted with substituents independently selected from hydroxyl, amino, alkyl, aryl, heteroaryl, or heterocyclyl; and
   n is 3 or 4;
   or a pharmaceutically acceptable salt, geometric isomer, enantiomer, diastereomer, racemate, prodrug, solvate, or hydrate thereof.

The compound of formula (II) is used as a starting compound for preparing the compound of formula (I).

In a further embodiment of the compound of formula (II), ring A is indazolyl; R₁ is selected from a group consisting of

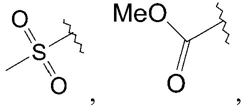

-CH₃,

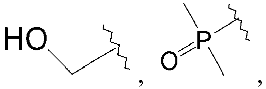

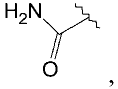

-CN, -OH,

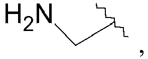

-OCH₃, -F, -Cl, -Br,

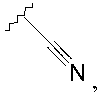

-CF₃, -CCl₃, -NO₂,

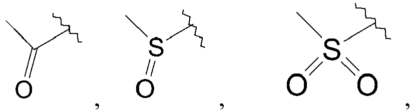

-B(OH)₂,

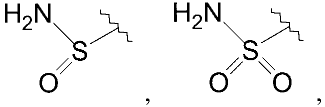

and 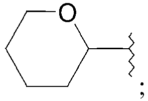 and n is 3 or 4.

In a preferred embodiment, the present disclosure provides a compound of formula (II) selected from:

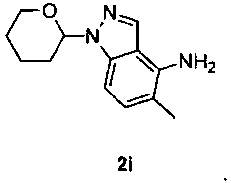

### Pharmaceutical composition, use and method

The compounds of the present disclosure may be therapeutically administered as pure chemicals, but it may be suitable to administer the compounds as pharmaceutical compositions or formulations. Accordingly, the present disclosure provides a pharmaceutical composition, which includes a therapeutically effective amount of a compound of formula (I) or a pharmaceutically acceptable salt, geometric isomer, enantiomer, diastereomer, racemate, prodrug, solvate, or hydrate thereof, and one or more pharmaceutically acceptable carriers or excipients.

The pharmaceutical composition can be administered in a variety of dosage forms, including, but not limited to: solid dosage form or liquid dosage form, oral dosage form, parenteral dosage form, intranasal dosage form, suppository, buccal lozenge, sugar-coated lozenge, oral dosage form, controlled-release dosage form, pulsatile-release dosage form, immediate-release dosage form, intravenous solution, suspension or a combination thereof. The compound may be administered, for example, by oral or parenteral routes, including intravenously, intramuscularly, intraperitoneally, subcutaneously, transdermally, via the respiratory tract (aerosol), rectally, vaginally and topically (including orally and sublingually).

In one embodiment of the present disclosure, the compound of formula (I) is orally administered. For oral administration, typically the compound will be provided as a tablet, pill, sugar-coated pill, buccal lozenge or capsule; as powder or granule; or as an aqueous solution, suspension, liquid, gel, syrup, slurry, and the like in a unit dosage form suitable for ingestion by an individual. The dosage form may be a tablet or a controlled-release dosage form formulated with a tablet. The tablet for oral use may include active ingredients mixed with one or more pharmaceutically acceptable excipients.

"Excipient" generally means a substance, usually an inert substance, added to a pharmacological composition or additionally used as a vehicle to further assist in the administration of the compound. Examples of the excipients include, but are not limited to: inert diluent, disintegrant, binder, lubricant, sweetener, flavor, colorant, preservative, foaming mixture and adsorbent. A suitable inert diluent includes, but is not limited to, sodium carbonate and calcium carbonate, sodium phosphate and calcium phosphate, lactose, and the like. A suitable disintegrant includes, but is not limited to: starch (e.g., corn starch), cross-linked polyvinylpyrrolidone, agar, alginic acid or salts thereof (e.g., sodium alginate), and the like. The binder may include, but is not limited to: magnesium aluminum silicate, starch (e.g., corn, wheat or rice starch), gelatin, methylcellulose, sodium carboxymethyl cellulose, polyvinylpyrrolidone, and the like. If present, the lubricant is typically magnesium stearate and calcium stearate, stearic acid, talc or hydrogenated vegetable oil. If desired, the tablet may be coated with a material such as glycerol monostearate or glycerol distearate to delay absorption in the gastrointestinal tract. The composition may also be formulated into chewable tablets, for example, by using a substance such as mannitol during the formulation.

The pharmaceutical composition for oral administration may be obtained by combining the compound of formula (I) with a solid excipient, optionally milling the resulting mixture, and processing the mixed particles to obtain tablets or cores for sugar-coated pills after the addition of other suitable compounds (if desired). A suitable solid excipient, in addition to those previously mentioned, includes carbohydrate or protein filler including, but not limited to: sugar, including lactose, sucrose, mannitol, or sorbitol; starch from corn, wheat, rice, potato, or other plants; cellulose, such as methylcellulose, hydroxypropyl methylcellulose, or sodium carboxymethyl cellulose; and gum, including arabic gum and tragacanth gum; and protein, such as gelatin and collagen.

The capsule for oral administration includes a hard gelatin capsule in which the active ingredients are mixed with a solid diluent, and a soft gelatin capsule in which the active ingredients are mixed with water or oil (e.g., peanut oil, liquid paraffin or olive oil).

The cores for sugar-coated pills have suitable coatings. For this purpose, a concentrated sugar solution may be used, which may optionally contain arabic gum, talc, polyvinylpyrrolidone, carbopolgel, polyethylene glycol and/or titanium dioxide, lacquer and a suitable organic solvent or a solvent mixture. A dye or pigment may be added to the tablets or a coating for sugar-coated pills to identify or characterize different combinations of active compound doses.

The pharmaceutical composition may also include a suitable solid or gel phase carrier. Examples of such carriers include, but are not limited to: calcium carbonate, calcium phosphate, various sugars, starch, cellulose derivative, gelatin, and polymer such as polyethylene glycol.

The compound and pharmaceutical composition suitable for use in the present disclosure include formulations in which the active ingredients are administered in an amount effective to achieve their intended purpose. The term "therapeutically effective amount" means an amount of the compound of formula (I) or a pharmaceutically acceptable salt, geometric isomer, enantiomer, diastereomer, racemate, prodrug, solvate, or hydrate thereof, alone or in combination with ionizing radiation or an anticancer agent, that provides a desired effect in a treated individual when administered to the individual in one or more doses. The toxicity and therapeutic efficacy of such compounds can be determined in cell cultures or experimental animals by standard pharmaceutical steps, e.g., by measuring IC₅₀ values. As used herein, "IC₅₀" means the concentration of an agent at which the agent is likely to produce 50% of the maximum inhibitory response.

The actual amount of the compound of formula (I) to be administered will be determined by the practitioner under relevant conditions, including the condition to be treated, the size and type of neoplasia, the route of administration as selected, the actual compound of the present disclosure as administered, the timing of administration of the hedgehog pathway modulator relative to other therapies, the type, species, age, weight, sex and medical condition of the individual, the renal and hepatic function of the individual, and the symptom severity of the individual. Achieving optimal accuracy in producing drug concentrations in the efficacy range requires protocols based on the kinetics of drug availability to the target site. This involves considering the distribution, equilibrium and elimination of the drug. In some cases, dose levels below the lower limit of the preceding range may be more than the full dose, while in other cases larger doses may still be used.

An "individual" to be treated by the method of the present disclosure means a human or non-human animal, such as a primate, mammal or vertebrate.

"In vivo" means within a living individual, such as an animal or a human being. As used herein, an agent may be used therapeutically in vivo to retard or eliminate the proliferation of abnormally replicating cells. The agent may also be used in vivo as a prophylactic agent to prevent the abnormal cell proliferation or the manifestation of symptoms associated with this.

"In vitro" means outside a living individual. Examples of in vitro cell populations include cell culture and biological samples, such as fluid or tissue samples from humans or animals. Such samples may be obtained by methods well known in the field to which they belong. Exemplary biofluid samples include blood, cerebrospinal fluid, urine and saliva. Exemplary tissue samples include tumors and biopsy thereof. As used herein, the compound of the present disclosure may be applied to a wide variety of applications, including both therapeutic applications and experimental applications.

The present disclosure also relates to use of the compound of formula (I), or a pharmaceutically acceptable salt, geometric isomer, enantiomer, diastereomer, racemate, prodrug, solvate, or hydrate thereof in the manufacture of a medicament for the modulation of the hedgehog pathway.

Additionally, the present disclosure relates to a method for treating a cancer in an individual in need thereof, which includes administering to the individual a therapeutically effective amount of the compound of formula (I) or a pharmaceutically acceptable salt, geometric isomer, enantiomer, diastereomer, racemate, prodrug, solvate, or hydrate thereof.

The "cancer" refers to a cell proliferative disease state that includes, but is not limited to: cardiac cancers, such as sarcoma (e.g., angiosarcoma, fibrosarcoma, rhabdomyosarcoma, and liposarcoma), myxoma, rhabdomyoma, fibroma, lipoma and teratoma; lung cancers, such as bronchogenic carcinoma (e.g., squamous cell carcinoma, undifferentiated small cell carcinoma, undifferentiated large cell carcinoma, and adenocarcinoma), alveolar carcinoma (e.g., bronchiolar carcinoma), bronchial adenoma, sarcoma, lymphoma, chondromatous hanlartoma, and inesothelioma; gastrointestinal cancers, such as esophagus carcinoma (e.g., squamous cell carcinoma, adenocarcinoma, leiomyosarcoma, and lymphoma), stomach carcinoma (e.g., lymphoma, and leiomyosarcoma), pancreas carcinoma (e.g., ductal adenocarcinoma, insulinoma, glucagonoma, gastrinoma, carcinoid, and vipoma), small bowel carcinoma (e.g., adenocarcinoma, lymphoma, carcinoid, Karposi's sarcoma, leiomyoma, hemangioma, lipoma, neurofibroma, and fibroma), large bowel carcinoma (e.g., adenocarcinoma, tubular adenoma, villous adenoma, hamartoma, and leiomyoma); genitourinary tract cancers, such as kidney carcinoma (e.g., adenocarcinoma, Wilm's tumor [nephroblastoma], lymphoma, and leukemia), bladder and urethra carcinoma (e.g., squamous cell carcinoma, transitional cell carcinoma, and adenocarcinoma), prostate carcinoma (e.g., adenocarcinoma, and sarcoma), testis carcinoma (e.g., seminoma, teratoma, embryonal carcinoma, teratocarcinoma, choriocarcinoma, sarcoma, interstitial cell carcinoma, fibroma, fibroadenoma, adenomatoid tumors, and lipoma); liver cancers, such as hepatoma (e.g., hepatocellular carcinoma), cholangiocarcinoma, hepatoblastoma, angiosarcoma, hepatocellular adenoma, and hemangioma; bone cancers, such as osteogenic sarcoma (e.g., osteosarcoma), fibrosarcoma, malignant fibrous histiocytoma, chondrosarcoma, Ewing's sarcoma, malignant lymphoma (e.g., reticulum cell sarcoma), multiple myeloma, malignant giant cell tumor chordoma, osteochronfroma (e.g., osteocartilaginous exostoses), benign chondroma, chondroblastoma, chondromyxofibroma, osteoid osteoma, and giant cell tumors; nervous system cancers, such as skull carcinoma (e.g., osteoma, hemangioma, granuloma, xanthoma, and osteitis deformans), meninx carcinoma (e.g., meningioma, meningiosarcoma, and gliomatosis), brain carcinoma (e.g., astrocytoma, medulloblastoma, glioma, ependymoma, germinoma (pinealoma), glioblastoma multiform, oligodendroglioma, schwannoma, retinoblastoma, and congenital tumors), spinal cord neurofibroma, meningioma, glioma, and sarcoma; gynecological cancers, such as uterus carcinoma (e.g., endometrial carcinoma), cervix carcinoma (e.g., cervical carcinoma, and pre-tumor cervical dysplasia), ovary carcinoma (e.g., ovarian carcinoma [e.g., serous cystadenocarcinoma, mucinous cystadenocarcinoma, and unclassified carcinoma], granulosa-thecal cell tumors, Sertoli-Leydig cell tumors, dysgerminoma, and malignant teratoma), vulva carcinoma (e.g., squamous cell carcinoma, intraepithelial carcinoma, adenocarcinoma, fibrosarcoma, and melanoma), vagina carcinoma (e.g., clear cell carcinoma, squamous cell carcinoma, botryoid sarcoma (e.g., embryonal rhabdomyosarcoma], and fallopian tube carcinoma); hematologic cancers, such as blood carcinoma (myeloid leukemia [acute and chronic], acute lymphoblastic leukemia, chronic lymphocytic leukemia, myeloproliferative diseases, multiple myeloma, and myelodysplastic syndrome), Hodgkin's disease, non-Hodgkin's lymphoma [malignant lymphoma]; skin cancers, such as malignant melanoma, basal cell carcinoma, squamous cell carcinoma, Karposi's sarcoma, moles dysplastic nevi, lipoma, angioma, dermatofibroma, keloids, psoriasis; adrenal gland cancers, such as neuroblastoma; or breast cancer.

The compound of formula (I) can be administered as the sole active agent, or in combination with other known cancer treatments.

The term "in combination with" means that the compound of formula (I) may be administered shortly before, shortly after, concurrently, or any combination of shortly before, shortly after or concurrently, with one or more other anti-tumor therapies. Thus, the compound of formula (I) and a second anticancer agent may be administered either as a single composition or simultaneously as two separate compositions or sequentially as two separate compositions. Likewise, the compound of formula (I) and a chemotherapy or ionizing radiation therapy may be administered simultaneously, separately or sequentially. Those skilled in the art will recognize that the amount of the compound of formula (I) to be administered in combination with anticancer therapies is preferably that amount sufficient to enhance the effect of the anticancer therapies or that amount sufficient to induce apoptosis or cell death along with the anticancer therapies to maintain an antiangiogenic effect.

The term "second anticancer agent" as used herein, unless otherwise indicated, means an agent capable of inhibiting or preventing the growth of tumors, or examining the maturation and proliferation of malignant (cancer) cells. The second anticancer agent suitable for use in combination with the compound of formula (I) includes, but is not limited to: targeted cancer drugs, such as trastuzumab, ramucirumab, bevacizumab, everolimus, tamoxifen, toremifene, fulvestrant, anastrozole, exemestane, lapatinib, letrozole, pertuzumab, ado-trastuzumab emtansine, palbociclib, cetuximab, panitumumab, ziv-aflibercept, regorafenib, lmatinib mesylate, lanreotide acetate, sunitinib, regorafenib, denosumab, alitretinoin, sorafenib, pazopanib, temsirolimus, everolimus, tretinoin, dasatinib, nilotinib, bosutinib, rituximab, alemtuzumab, ofatumumab, obinutuxumab, ibrutinib, idelalisib, blinatumomab, soragenib, crizotinib, erlotinib, gefitinib, afatinib dimaleate, ceritnib, ramucirumab, nivolumab, pembrolizumab, osimertinib, and necitumumab; alkylating agents, such as busulfan, chlorambucil, cyclophosphamide, iphosphamide, melphalan, nitrogen mustard, streptozocin, thiotepa, uracil nitrogen mustard, triethylenemelamine, temozolomide, and 2-chloroethyl-3-sarcosinamide-1-nitrosourea (SarCNU); antibiotics or plant alkaloids, such as actinomycin-D, bleomycin, cryptophycin, daunorubicin, doxorubicin, idarubicin, irinotecan, L-asparaginase, mitomycin-C, mitramycin, navelbine, paclitaxel, docetaxel, topotecan, vinblastine, vincristine, teniposide (VM-26), and etoposide (VP-16); hormones or steroids, such as 5α-reductase inhibitor, aminoglutethimide, anastrozole, bicalutamide, chlorotrianisene, diethylstilbestrol (DES), dromostanolone, estramustine, ethinyl estradiol, flutamide, fluoxymesterone, goserelin, hydroxyprogesterone, letrozole, leuprolide, medroxyprogesterone acetate, megestrol acetate, methyl prednisolone, methyltestosterone, mitotane, nilutamide, prednisolone, arzoxifene (SERM-3), tamoxifen, testolactone, testosterone, triamicnolone, and zoladex; synthetics, such as all-trans retinoic acid, carmustine (BCNU), carboplatin (CBDCA), lomustine (CCNU), cis-diaminedichloroplatinum (cisplatin), dacarbazine, gliadel, hexamethylmelamine, hydroxyurea, levamisole, mitoxantrone, o,ρ'-dichlorodiphenyldichloroethane (o,ρ'-DDD) (also known as lysodren or mitotane), oxaliplatin, porfimer sodium, procarbazine, and imatinib mesylate (Gleevec^{®}); antimetabolites, such as chlorodeoxyadenosine, cytosine arabinoside, 2'-deoxycoformycin, fludarabine phosphate, 5-fluorouracil (5-FU), 5-fluoro-2'-deoxyuridine (5-FUdR), gemcitabine, camptothecin, 6-mercaptopurine, methotrexate, and thioguanine; and biologics, such as alpha interferon, BCG (Bacillus Calmette-Guerin), granulocyte colony stimulating factor (G-CSF), granulocyte-macrophage colony-stimulating factor (GM-CSF), interleukin-2, and herceptin.

The term "treating" as used herein, unless otherwise indicated, means reversing, alleviating and inhibiting a disorder or condition to which such term applies or the development of one or more symptoms of such disorder or condition, or preventing the disorder or condition or one or more symptoms of such disorder or condition. The term "treatment" as used herein, unless otherwise indicated, refers to the act of treating as defined above.

### Compound Synthesis

The present disclosure also relates to a process for preparing a compound of formula (I) or a pharmaceutically acceptable salt, geometric isomer, enantiomer, diastereomer, racemate, prodrug, solvate, or hydrate thereof. The compound of the present disclosure can be prepared by a person skilled in the art using conventional organic synthesis methods and commercially available materials.

In one embodiment, the process for preparing a compound of formula (I) or a pharmaceutically acceptable salt, geometric isomer, enantiomer, diastereomer, racemate, prodrug, solvate, or hydrate thereof comprises:
a. reacting a compound of formula (II) 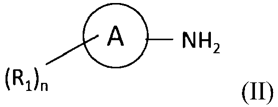 with a pyridine of formula (III) 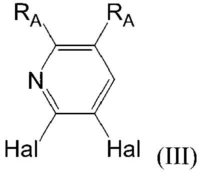 in the presence of a catalyst to obtain a compound of formula (IV) 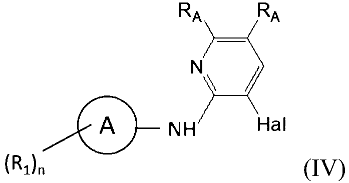 wherein ring A, R₁, and n are as defined in the specification, Hal is halogen, and R_{A} is alkyl;
b. reacting the compound of formula (IV) with a coupling agent containing cyano to obtain a compound of formula (I') 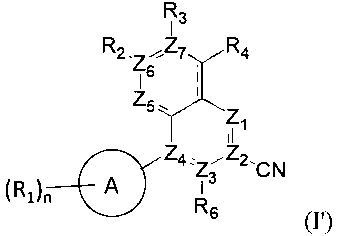 wherein ring A, Z₁, Z₂, Z₃, Z₄, Z₅, Z₆, Z₇, R₁, R₂, R₃, R₄, R₆, and n are as defined in the specification;
c. allowing the compound of formula (I') to undergo an acylation reaction in the presence of an acid to obtain the compound of formula (I) 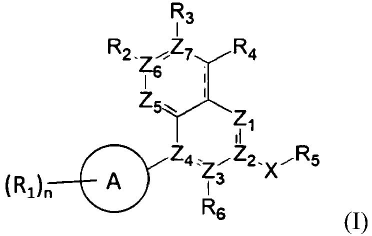 wherein ring A, Z₁, Z₂, Z₃, Z₄, Z₅, Z₆, Z₇, X, R₁, R₂, R₃, R₄, R₅, R₆, and n are as defined in the specification.

By way of example, but not intended to be limiting, the general synthesis of representative compounds of formula (I) or a pharmaceutically acceptable salt, geometric isomer, enantiomer, diastereomer, racemate, prodrug, solvate, or hydrate thereof is shown in Scheme 1. It should be noted that those skilled in the art can modify the steps outlined in the illustrative flow charts and examples to obtain the desired product.

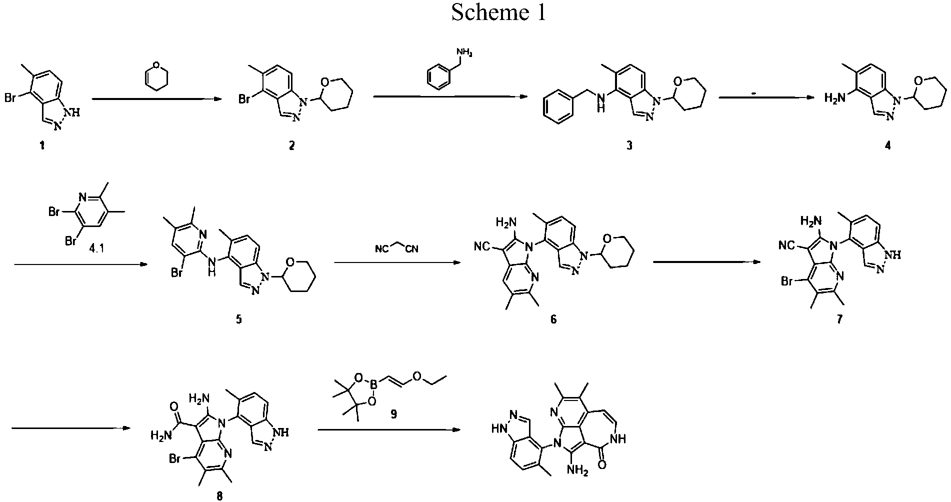

### EXAMPLE

### Example 1

### Synthesis of compound 126

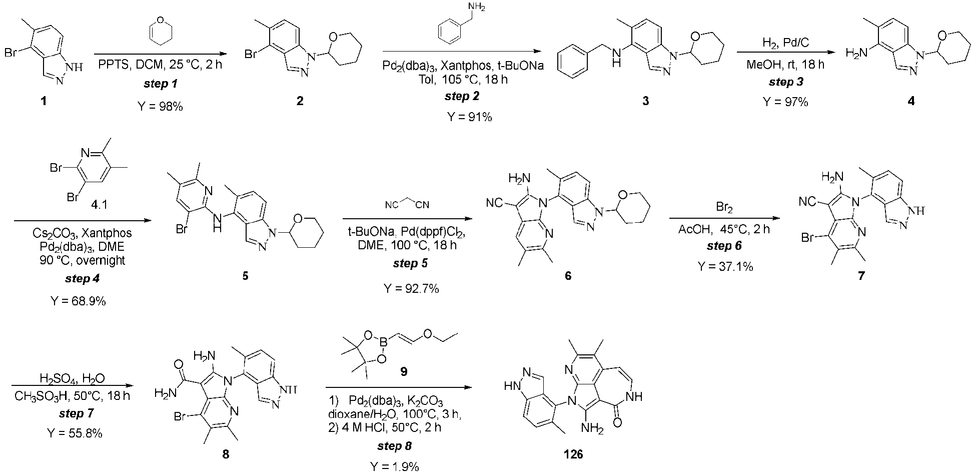

### Step 1

To a solution of compound **126-1** (50 g, 236.90 mmol) and 3,4-dihydro-2H-pyran (43.32 mL, 473.80 mmol, Anji) in dichloromethane (400 mL) was added p-toluenesulfonic acid monohydrate (4.51 g, 23.69 mmol). The resulting mixture was stirred at room temperature for 2 hours. The reaction mixture was then concentrated to dryness to obtain a crude product. The crude product was purified by silica gel chromatography (eluent: petroleum ether: ethyl acetate = 20:1 to 10:1) to afford compound **126-2** as a yellow solid (68.4 g, 231.72 mmol, yield = 98%). LC-MS (ESI): m/z = 295.0/297.0 [M+H]⁺.

### Step 2

To a mixed solution of compound **126-2** (40 g, 135.51 mmol), benzylamine (15.56 mL, 142.29 mmol), sodium *tert*-butoxide (26.05 g, 271.02 mmol), 4,5-bis(diphenylphosphino)-9,9-dimethylxanthene (4.70 g, 8.13 mmol), and toluene (400 mL), tris(dibenzylideneacetone)dipalladium(0) (3.72 g, 4.07 mmol) were added. The mixture was degassed and purged with nitrogen three times, then stirred at 105°C for 18 hours. The reaction mixture was concentrated to dryness to yield a crude product. The crude product was purified by silica gel chromatography (eluent: petroleum ether:ethyl acetate = 10:1 to 3:1) to afford compound **126-3** as a yellow solid (39.8 g, 123.83 mmol, yield = 91%). LC-MS (ESI): m/z = 322.2 [M+H]⁺.

### Step 3

To a mixed solution of compound **126-3** (21.1 g, 65.65 mmol) in methanol (200 mL) and acetic acid (0.5 mL), palladium on carbon (3.49 g, 3.28 mmol) was added. The mixture was degassed and purged with hydrogen three times, then stirred at room temperature for 18 hours. The mixture was filtered, and the filter cake was washed three times with methanol (200 mL). The combined filtrate was concentrated to dryness to afford compound **126-4** as a gray solid (15.46 g, 63.50 mmol, yield = 97%). LC-MS (ESI): m/z = 232.1 [M+H]⁺.

### Step 4

To a mixed solution of compound **126-4** (2.00 g, 7.55 mmol), compound **126-4.1** (1.75 g, 7.55 mmol), cesium carbonate (6.15 g, 18.87 mmol), and 1,2-dimethoxyethane (40 mL), tris(dibenzylideneacetone)dipalladium(0) (0.69 g, 0.75 mmol) and 4,5-bis(diphenylphosphino)-9,9-dimethylxanthene (0.87 g, 1.51 mmol) were added separately. The reaction mixture was purged with nitrogen three times and stirred at 90°C for 18 hours. The reaction mixture was concentrated to dryness to yield a crude product. The crude product was purified by silica gel chromatography (eluent: petroleum ether:ethyl acetate = 20:1 to 10:1) to afford compound **126-5** as a yellow solid (2.16 g, 5.20 mmol, yield = 68.9%). LC-MS (ESI): m/z = 415.2/417.2 [M+H]⁺.

### Step 5

To a mixed solution of compound **126-5** (2.16 g, 5.20 mmol), malononitrile (1.03 g, 15.60 mmol, freshly distilled), and 1,2-dimethoxyethane (40 mL), 1,1'-bis(diphenylphosphino)ferrocene palladium dichloride (0.38 g, 0.52 mmol) and sodium tert-butoxide (1.25 g, 13.00 mmol) were added separately. The reaction mixture was purged with nitrogen three times and stirred at 100°C for 18 hours. The reaction mixture was concentrated to dryness to yield a crude product. The crude product was purified by silica gel chromatography (eluent: ethyl acetate:petroleum ether = 30-40%) to afford compound **126-6** as a yellow solid (1.93 g, 4.82 mmol, yield = 92.7%). LC-MS (ESI): m/z = 401.2 [M+H]⁺.

### Step 6

To a solution of compound **126-6** (900 mg, 2.25 mmol) in acetic acid (18 mL), liquid bromine (230.95 µL, 4.49 mmol) was added. The mixture was stirred at 45°C for 2 hours. The mixture was poured into water (50 mL) and extracted three times with ethyl acetate (30 mL). The combined organic layers were washed with brine (50 mL), dried over anhydrous sodium sulfate, filtered, and concentrated under reduced pressure to dryness to yield a crude product. The crude product was purified by reverse-phase preparative chromatography (eluent: water:acetonitrile = 3:2) to afford compound **126-7** as a yellow solid (330 mg, 0.83 mmol, yield = 37.1%). LC-MS (ESI): m/z = 395.2/397.2 [M+H]⁺.

### Step 7

To a solution of compound **126-7** (120 mg, 0.30 mmol) in methanesulfonic acid (2 mL), water (180 µL, 9.99 mmol) and concentrated sulfuric acid (90 µL, 1.70 mmol) were added. The mixture was stirred at 50°C for 18 hours. The mixture was adjusted to pH = 8 with 1 M sodium hydroxide solution and extracted three times with ethyl acetate (10 mL). The combined organic layers were washed with saturated brine (30 mL), dried over anhydrous sodium sulfate, filtered, and concentrated to dryness to yield a crude product. The crude product was purified by reverse-phase preparative chromatography (eluent: 30% to 40% (v/v) acetonitrile in water containing 0.1% trifluoroacetic acid) to afford compound **126-8** as a yellow solid (70 mg, 0.17 mmol, yield = 55.8%). LC-MS (ESI): m/z = 413.2/415.2 [M+H]⁺.

### Step 8

To a mixed solution of compound **126-8** (70 mg, 0.17 mmol), compound **126-9** (53.53 µL, 0.26 mmol), 1,4-dioxane (2 mL), and water (0.5 mL), potassium carbonate (70 mg, 0.51 mmol), tricyclohexylphosphine (9.5 mg, 0.03 mmol), and tris(dibenzylideneacetone)dipalladium(0) (15.5 mg, 0.02 mmol) were added. The mixture was stirred under nitrogen at 100°C for 3 hours. Then, hydrochloric acid (382 µL, 1.53 mmol, 4 M) was added, and the mixture was stirred at 50°C for 2 hours. The mixture was concentrated to dryness, and the crude product was purified by preparative HPLC (Waters-SunFire-C18-10 µm, 19 × 250 mm; eluent: 29% to 59% (v/v) acetonitrile in water containing 10 mM ammonium bicarbonate) to afford compound **126** as a white solid (1.33 mg, 0.003 mmol, yield = 1.9%). LC-MS (ESI): m/z = 359.2 [M+H]⁺. ¹H NMR (400 MHz, DMSO-*d*₆) δ: 13.24 (s, 1H), 8.27 (d, *J* = 7.0 Hz, 1H), 7.63 (d, *J* =8.6 Hz, 1H), 7.52 (d, *J* = 1.0 Hz, 1H), 7.41 (d, *J* = 8.6 Hz, 1H), 6.47 (s, 2H), 5.64 (dd, *J=* 10.6, 7.2 Hz, 1H), 5.24 (d, *J* = 10.2 Hz, 1H), 2.08 (s, 3H), 2.04 (s, 3H), 1.98 (s, 3H).

### Example 2

### Synthesis of compound 136

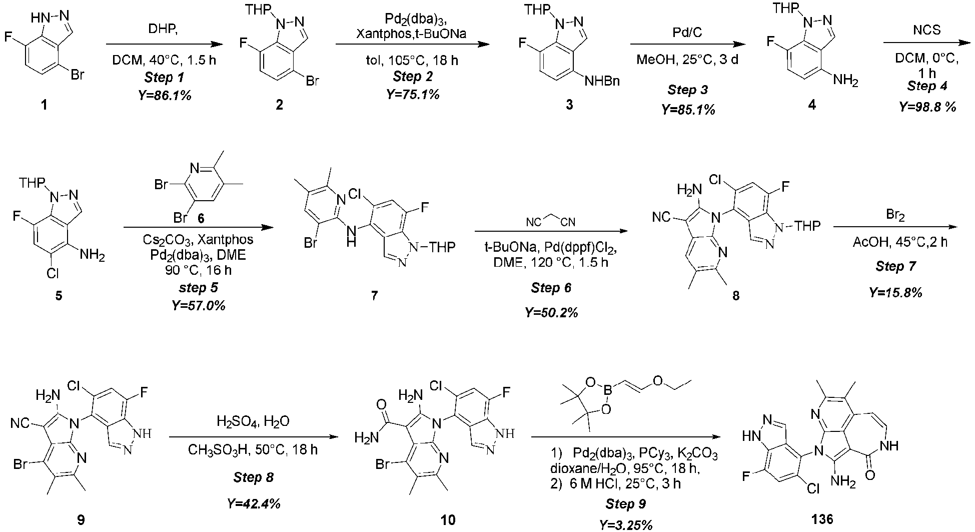

### Step 1

To a solution of compound **136-1** (5 g, 23.25 mmol, BidePharma) and 3,4-dihydro-2H-pyran (4.25 mL, 46.51 mmol) in dichloromethane (100 mL), *p*-toluenesulfonic acid monohydrate (0.44 g, 2.33 mmol) was added. The resulting mixture was stirred at 40°C for 1.5 hours. The mixture was concentrated to dryness to yield a crude product. The crude product was purified by silica gel chromatography (eluent: petroleum ether:ethyl acetate = 20:1 to 10:1) to afford compound **136-2** as a white solid (6 g, 20.06 mmol, yield = 86.1%). LC-MS (ESI): m/z = 299.0/301.0 [M+H]⁺.

### Step 2

To a solution of compound **136-2** (6 g, 20.06 mmol) in toluene (100 mL), benzylamine (2.46 mL, 22.46 mmol), sodium tert-butoxide (6.17 g, 64.18 mmol), and 4,5-bis(diphenylphosphino)-9,9-dimethylxanthene (19.35 mg, 0.03 mmol) were added, followed by the addition of tris(dibenzylideneacetone)dipalladium(0) (7.65 mg, 0.01 mmol). The mixture was degassed and purged with N₂ three times, then stirred under nitrogen atmosphere at 105°C for 18 hours. The reaction mixture was poured into water (100 mL) and extracted with ethyl acetate (100 mL × 3). The combined organic layers were washed with brine (300 mL), dried over anhydrous sodium sulfate, filtered, and concentrated to dryness to yield a crude product. The crude product was purified by silica gel chromatography (eluent: petroleum ether:ethyl acetate = 10:1 to 2:1) to afford compound **136-3** as a brown solid (5.23 g, 16.07 mmol, yield = 75.1%). LC-MS (ESI): m/z = 326.2 [M+H]⁺.

### Step 3

To a methanol (100 mL) solution of compound **136-3** (5.23 g, 16.07 mmol), wet Pd/C (0.84 mL, 10%, 0.80 mmol) was added. The mixture was degassed and replaced with hydrogen gas three times, then stirred at 25°C for 3 days. The suspension was filtered through diatomaceous earth, washed with methanol (100 mL), and the filtrate was concentrated to dryness. The crude product was purified by silica gel chromatography (eluent: petroleum ether:ethyl acetate = 10:1 to 6:4) to afford compound **136-4** as a yellow oil (3.22 g, 13.69 mmol, yield = 85.1%). LC-MS (ESI): m/z = 236.2 [M+H]⁺.

### Step 4

To a dichloromethane (50 mL) solution of compound **136-4** (3.22 g, 13.69 mmol), N-chlorosuccinimide (2.01 g, 15.06 mmol) was added, and the mixture was stirred at 0°C for 1 hour. The mixture was poured into water (20 mL) and extracted with dichloromethane (30 mL) three times. The combined organic layers were washed with brine (50 mL), dried over anhydrous sodium sulfate, filtered, and concentrated to dryness to obtain the crude product. The crude product was purified by silica gel chromatography (eluent: petroleum ether:ethyl acetate = 10:1 to 5:1) to afford compound **136-5** as a yellow solid (3.65 g, 13.53 mmol, yield = 98.8%). LC-MS (ESI): m/z = 270.1 [M+H]⁺.

### Step 5

Compound **136-5** (3.65 g, 13.53 mmol), compound **136-6** (3.59 g, 13.53 mmol), and cesium carbonate (3.04 g, 9.34 mmol) were added to 1,2-dimethoxyethane (30 mL). Then, tris(dibenzylideneacetone)dipalladium(0) (0.68 g, 0.74 mmol) and 4,5-bis(diphenylphosphino)-9,9-dimethylxanthene (0.86 g, 1.48 mmol) were added separately. The mixture was degassed and purged with nitrogen three times, then stirred at 90°C for 16 hours. The mixture was poured into water (100 mL) and extracted with ethyl acetate (100 mL) three times. The combined organic layers were washed with brine (20 mL), dried over anhydrous sodium sulfate, filtered, and concentrated to dryness to obtain the crude product. The crude product was purified by silica gel chromatography (eluent: petroleum ether:ethyl acetate = 10:1 to 5:1) to afford compound **136-7** as a yellow solid (3.5 g, 7.71 mmol, yield = 57.0%). LC-MS (ESI): m/z = 453.2/455.2 [M+H]⁺.

### Step 6

Compound **136-7** (3.5 g, 7.71 mmol), propanedinitrile (2.04 g, 30.85 mmol, purchased), sodium tert-butoxide (2.22 g, 23.14 mmol), and 1,2-dimethoxyethane (25 mL) were added separately into a 40 mL microwave vial. The resulting mixture was purged with nitrogen for 5 minutes. Then, 1,1'-bis(diphenylphosphino)ferrocene palladium(II) dichloride (1.13 g, 1.54 mmol) was added. The mixture was stirred under microwave irradiation at 120°C for 1.5 hours. The mixture was poured into water (100 mL) and extracted with ethyl acetate (50 mL) three times. The combined organic layers were washed with brine (50 mL), dried over anhydrous sodium sulfate, filtered, and concentrated to dryness to obtain the crude product. The crude product was purified by silica gel chromatography (eluent: petroleum ether:ethyl acetate = 10:1 to 1:1) to afford compound **136-8** as a yellow oil (1700 mg, 3.87 mmol, yield = 50.2%). LC-MS (ESI): m/z = 439.2 [M+H]⁺.

### Step 7

Liquid bromine (117.08 µL, 2.28 mmol) was added to a mixture of compound **136-8** (900 mg, 2.25 mmol) and acetic acid (4 mL). The resulting mixture was stirred at 45°C for 2 hours. The mixture was poured into water (20 mL) and extracted with ethyl acetate (30 mL) three times. The combined organic layers were washed with brine (10 mL), dried over anhydrous sodium sulfate, filtered, and concentrated to dryness to obtain the crude product. The crude product was purified by preparative HPLC (Phenomenex Gemini 150 mm × 25 mm × 10 µm column; eluent: 30% to 60% (v/v) acetonitrile and water containing 0.025% formic acid) to afford compound **136-9** as a yellow solid (150 mg, 0.35 mmol, yield = 15.8%). LC-MS (ESI): m/z = 433.0/435.0 [M+H]⁺.

### Step 8

Compound **136-9** (102 mg, 0.24 mmol) was added to a solution of methanesulfonic acid (2 mL), followed by the addition of concentrated sulfuric acid (137.90 µL, 2.59 mmol) and water (55.10 µL, 3.06 mmol). The reaction mixture was stirred at 50°C for 18 hours. The mixture was quenched with saturated sodium bicarbonate solution (40 mL) and extracted with ethyl acetate (30 mL) three times. The combined organic layers were washed with brine (10 mL), dried over anhydrous sodium sulfate, filtered, and concentrated to dryness to obtain the crude product. The crude product was purified by silica gel chromatography (eluent: petroleum ether:ethyl acetate = 10:1 to 0:1) to afford compound **136-10** as a yellow oil (45 mg, 0.10 mmol, yield = 42.4%). LC-MS (ESI): m/z = 451.0/453.0 [M+H]⁺.

### Step 9

Compound **136-10** (35 mg, 0.08 mmol) and 2-[(1E)-2-ethoxyvinyl]-4,4,5,5-tetramethyl-1,3,2-dioxaborolane (32.83 µL, 0.15 mmol, BidePharma) were added separately to a dioxane (1 mL) solution. Then, tris(dibenzylideneacetone)dipalladium(0) (0.87 mg, 0.01 mmol, purchased), dicyclohexyl(2-(2,6-dimethoxyphenyl)phenyl)phosphane (3.18 mg, 0.01 mmol, purchased), and potassium carbonate (32.90 mg, 0.15 mmol) were added. The mixture was stirred under nitrogen at 95°C for 18 hours. Then, dilute HCl (2 mL, 6 mol/L) was added, and the mixture was stirred at 25°C for 3 hours. The mixture was poured into saturated sodium bicarbonate solution (20 mL) and extracted with ethyl acetate (30 mL) three times. The combined organic layers were washed with brine (10 mL), dried over anhydrous sodium sulfate, filtered, and concentrated to dryness to obtain the crude product. The crude product was purified by preparative HPLC (Phenomenex Gemini 150 mm × 25 mm × 10 µm column; eluent: 30% to 60% (v/v) acetonitrile and water containing 0.025% formic acid) to afford compound 136 as a white solid (1 mg, 0.001 mmol, yield = 3.25%). LC-MS (ESI): m/z = 396.9 [M+H]⁺. ¹H NMR (400 MHz, DMSO-*d*₆) δ: 14.10 (s, 1H), 8.35-8.26 (m, 1H), 7.98-7.81 (m, 1H), 7.71-7.63 (m, 1H), 6.71 (s, 2H), 5.73-5.58 (m, 1H), 5.26 (d, J = 10.4 Hz, 1H), 2.11 (s, 3H), 2.00 (s, 3H).

### Example 3

### Synthesis of compound 137

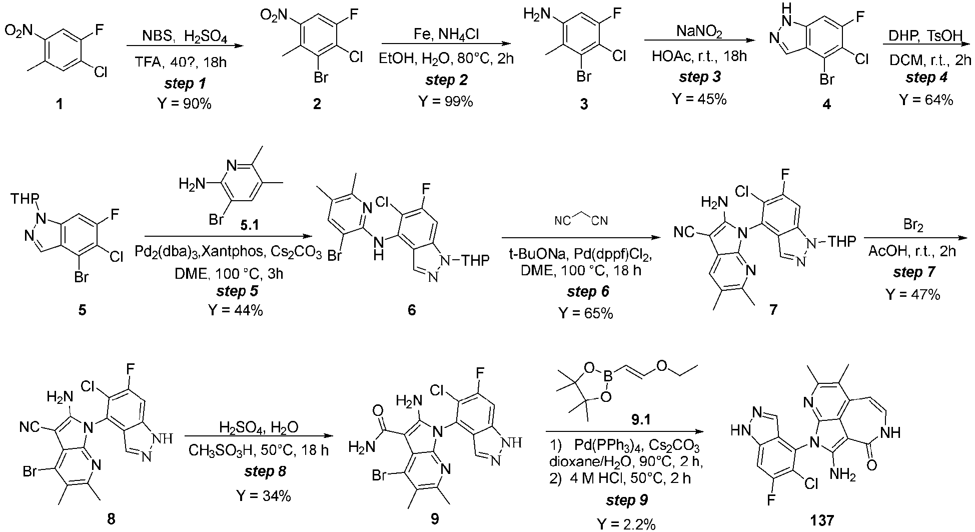

### Step 1

To a solution of compound **137-1** (2.2 g, 11.61 mmol, BidePharma) in trifluoroacetic acid (11 mL) and concentrated sulfuric acid (2.75 mL), N-bromosuccinimide (3.10 g, 17.41 mmol) was added. The mixture was stirred at 40°C for 18 hours. TLC analysis showed complete consumption of the starting material and the appearance of a less polar spot. The reaction mixture was poured into water (50 mL) and extracted with ethyl acetate (30 mL each) three times. The combined organic layers were washed with brine (50 mL), dried over anhydrous sodium sulfate, filtered, and concentrated to dryness to obtain the crude product. The crude product was purified by silica gel chromatography (eluent: 100% petroleum ether) to afford compound **137-2** as a green oil (2.8 g, 10.43 mmol, yield = 90%).

### Step 2

To a mixed solution of compound **137-2** (2.8 g, 10.43 mmol) in ethanol (50 mL) and water (10 mL), iron powder (2.33 g, 41.72 mmol) and ammonium chloride (1.67 g, 31.29 mmol) were added. The resulting mixture was stirred at 80°C for 2 hours. The mixture was filtered, the filter cake was washed with methanol (30 mL each) three times, and the filtrate was concentrated to dryness to obtain the crude product. The crude product was purified by silica gel chromatography (eluent: petroleum ether:ethyl acetate = 10:1 to 4:1) to afford compound **137-3** as a yellow oil (2.56 g, 10.41 mmol, yield = 99%). LC-MS (ESI): m/z = 238.0/240.0 [M+H]⁺.

### Step 3

At 0°C, sodium nitrite (2.38 g, 34.55 mmol) was added to a solution of compound **137-3** (2.06 g, 8.64 mmol) in acetic acid (20 mL). The solution was stirred at room temperature for 18 hours. The mixture was poured into water (50 mL) and extracted with ethyl acetate (50 mL each) three times. The combined organic layers were washed with brine (100 mL), dried over anhydrous sodium sulfate, filtered, and concentrated under reduced pressure to dryness to obtain the crude product. The crude product was purified by silica gel chromatography (eluent: petroleum ether:ethyl acetate = 10:1 to 4:1) to afford compound **137-4** as a yellow solid (1 g, 4.01 mmol, yield = 45%). LC-MS (ESI): m/z = 249.0/251.0 [M+H]⁺.

### Step 4

To a solution of compound **137-4** (1.0 g, 4.01 mmol) and 3,4-dihydro-2H-pyran (0.73 mL, 8.02 mmol) in dichloromethane (2 mL), *p*-toluenesulfonic acid monohydrate (76.25 mg, 0.40 mmol) was added. The mixture was stirred at room temperature for 2 hours. The mixture was concentrated to dryness to obtain the crude product. The crude product was purified by silica gel chromatography (eluent: petroleum ether:ethyl acetate = 10:1 to 5:1) to afford compound **137-5** as a yellow solid (868 mg, 2.60 mmol, yield = 64%). LC-MS (ESI): m/z = 333.0/335.0 [M+H]⁺.

### Step 5

To a solution of compound **137-5** (800 mg, 2.40 mmol) and compound 137-5.1 (337.54 mg, 1.68 mmol) in dimethoxyethane (16 mL), tris(dibenzylideneacetone)dipalladium(0) (219.61 mg, 0.24 mmol), 4,5-bis(diphenylphosphino)-9,9-dimethylxanthene (277.53 mg, 0.48 mmol), and cesium carbonate (1953.42 mg, 6.00 mmol) were added. The mixture was purged with nitrogen three times and stirred under nitrogen at 100°C for 3 hours. The mixture was poured into water (50 mL) and extracted with ethyl acetate (30 mL each) three times. The combined organic layers were washed with brine (100 mL), dried over anhydrous sodium sulfate, filtered, and concentrated under reduced pressure to dryness to obtain the crude product. The crude product was purified by silica gel chromatography (eluent: petroleum ether:ethyl acetate = 10:1 to 4:1) to afford compound **137-6** as a yellow solid (480 mg, 1.06 mmol, yield = 44%). LC-MS (ESI): m/z = 453.2/455.2 [M+H]⁺.

### Step 6

To a solution of compound **137-6** (480 mg, 1.06 mmol) and propanedinitrile (209.65 mg, 3.17 mmol, purchased) in dimethoxyethane (9 mL), 1,1'-bis(diphenylphosphino)ferrocene palladium(II) dichloride (77.4 mg, 0.10 mmol) and sodium tert-butoxide (254.15 mg, 2.65 mmol) were added. The mixture was purged with nitrogen three times and stirred under nitrogen at 120°C for 48 hours. The mixture was poured into water (40 mL) and extracted with ethyl acetate (30 mL each) three times. The combined organic layers were washed with brine (50 mL), dried over anhydrous sodium sulfate, filtered, and concentrated to dryness to obtain the crude product. The crude product was purified by silica gel chromatography (eluent: petroleum ether:ethyl acetate = 10:1 to 3:2) to afford compound **137-7** as a yellow solid (300 mg, 0.68 mmol, yield = 65%). LC-MS (ESI): m/z = 439.2 [M+H]⁺.

### Step 7

To a solution of compound **137-7** (300 mg, 0.68 mmol) in acetic acid (2 mL), liquid bromine (23.42 µL, 0.46 mmol) was added. The mixture was stirred at room temperature for 2 hours. The mixture was poured into water (20 mL) and extracted with ethyl acetate (20 mL each) three times. The combined organic layers were washed with brine (50 mL), dried over anhydrous sodium sulfate, filtered, and concentrated to dryness to obtain the crude product. The crude product was purified by reverse-phase preparative chromatography (eluent: 40% to 60% (v/v) acetonitrile in water containing 0.1% trifluoroacetic acid) to afford compound **137-8** as a yellow solid (140 mg, 0.32 mmol, yield = 47%). LC-MS (ESI): m/z = 433.0/435.0 [M+H]+.

### Step 8

To a solution of compound **137-8** (140 mg, 0.32 mmol) in methanesulfonic acid (1.4 mL), water (220 µL, 12.21 mmol) and concentrated sulfuric acid (110 µL, 2.06 mmol) were added. The mixture was stirred at 50°C for 18 hours. The reaction mixture was adjusted to pH = 8 with a saturated sodium hydroxide solution, then extracted with ethyl acetate (20 mL each) three times. The combined organic layers were washed with brine (30 mL), dried over anhydrous sodium sulfate, filtered, and concentrated to dryness to obtain the crude product. The crude product was purified by reverse-phase preparative chromatography (eluent: 40% to 50% (v/v) acetonitrile in water containing 0.1% trifluoroacetic acid) to afford compound **137-9** as a yellow solid (50 mg, 0.11 mmol, yield = 34%). LC-MS (ESI): m/z = 451.0/453.0 [M+H]⁺.

### Step 9

To a solution of compound **137-9** (40 mg, 0.09 mmol) and compound **137-9.1** (26.31 mg, 0.13 mmol) in 1,4-dioxane (2 mL) and water (0.4 mL), cesium carbonate (86.56 mg, 0.27 mmol) and tetrakis(triphenylphosphine)palladium(0) (10.24 mg, 0.01 mmol) were added. The mixture was stirred under nitrogen at 90°C for 2 hours. Then, hydrochloric acid (6 mL, 1.08 mmol, 4 M) was added and the mixture was stirred at 50°C for 2 hours. The mixture was adjusted to pH = 7 with 7 M ammonia in methanol solution and concentrated to dryness. The crude product was purified by preparative HPLC (Waters-SunFire-C18-10 µm, 19 × 250 mm; eluent: 37% to 67% (v/v) acetonitrile in water containing 0.1% formic acid) to afford compound **137** as a white solid (0.76 mg, 0.002 mmol, yield = 2.2%). LC-MS (ESI): m/z = 397.2 [M+H]⁺. LC-MS (ESI): m/z = 397.2 [M+H]⁺. ¹H NMR (400 MHz, DMSO-*d*₆) δ: 13.61 (s, 1H), 8.31 (d, *J =* 7.2 Hz, 1H), 7.84 (dd, *J =* 8.8, 1.2 Hz, 1H), 7.74 (d, *J* = 1.2 Hz, 1H), 6.77 (s, 2H), 5.65 (dd, *J =* 10.6, 7.2 Hz,1H), 5.25 (d, *J =* 10.6 Hz, 1H), 2.09 (s, 3H), 1.98 (s, 3H).

### Example 4

### Synthesis of compound 138

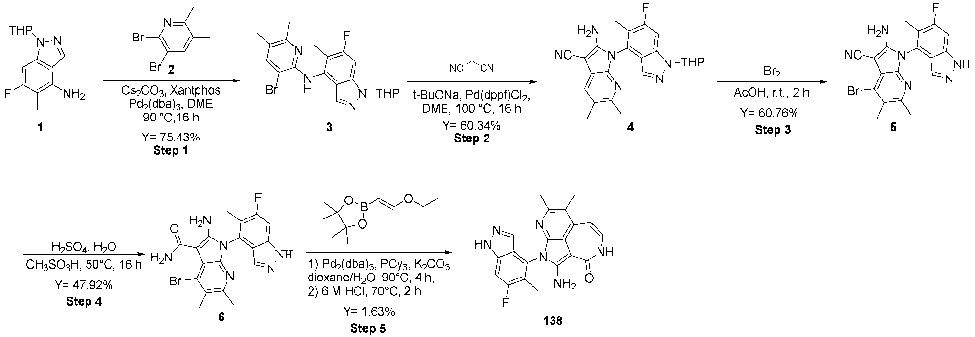

### Step 1

To a solution of compound **138-1** (900 mg, 3.61 mmol) in 8 mL of 1,2-dimethoxyethane (DME), compound **138-2** (1052.19 mg, 3.97 mmol), cesium carbonate (2940.73 mg, 9.03 mmol), tris(dibenzylideneacetone)dipalladium(0) (Pd₂(dba)₃) (330.60 mg, 0.36 mmol), and 4,5-bis(diphenylphosphino)-9,9-dimethylxanthene (Xantphos) (417.80 mg, 0.72 mmol) were added. The mixture was degassed and purged with nitrogen three times, then stirred under nitrogen atmosphere at 90°C for 16 hours. The mixture was concentrated under reduced pressure to dryness to obtain the crude product. The crude product was purified by silica gel chromatography (eluent: ethyl acetate : petroleum ether = 0% to 20%) to afford compound **138-3** as a yellow oil (1.18 g, 2.72 mmol, yield = 75.43%). LC-MS (ESI): m/z 433.2/435.2 [M+H]⁺.

### Step 2

To a solution of compound **138-3** (300 mg, 0.69 mmol) in 5 mL of 1,2-dimethoxyethane, bis[5-(diphenylphosphino)cyclopentadienyl]iron(II) palladium(II) chloride (50.66 mg, 0.07 mmol), malononitrile (137.2 mg, 2.08 mmol), and sodium 2-methylprop-2-enoate (166.33 mg, 1.73 mmol) were added. The mixture was degassed and purged with nitrogen three times, then stirred under nitrogen atmosphere at 100°C for 16 hours. The reaction mixture was poured into 4 mL of water and extracted with 4 mL of ethyl acetate three times. The combined organic layers were washed with 4 mL of brine, dried over anhydrous sodium sulfate, filtered, and concentrated under reduced pressure to dryness to obtain the crude product. The crude product was purified by silica gel chromatography (eluent: petroleum ether: ethyl acetate = 100:1 to 60:40) to afford compound **138-4** as a yellow oil (525 mg, 0.42 mmol, yield = 60.34%). LC-MS (ESI): m/z 419.2 [M+H]⁺.

### Step 3

To a solution of compound **138-4** (100 mg, 0.24 mmol) in 1 mL of acetic acid, liquid bromine (24.56 µL, 0.48 mmol) was added. The mixture was stirred at room temperature for 2 hours. The mixture was poured into 4 mL of water and extracted with 4 mL of ethyl acetate three times. The combined organic layers were washed with 4 mL of brine, dried over anhydrous sodium sulfate, filtered, and concentrated under reduced pressure to dryness to obtain the crude product. The crude product was purified by preparative HPLC (eluent: 0% to 70% (v/v) acetonitrile in water containing 0.025% ammonium bicarbonate) to afford compound **138-5** as a yellow solid (60 mg, 0.15 mmol, yield = 60.76%). LC-MS (ESI): m/z 413.0/415.0 [M+H]⁺.

### Step 4

To a solution of compound **138-5** (40 mg, 0.1 mmol) in 0.5 mL of methanesulfonic acid, water (61.05 µL, 3.39 mmol) and concentrated sulfuric acid (30.95 µL, 0.58 mmol) were added. The mixture was stirred at 50°C for 16 hours. The mixture was adjusted to a pH of 7 with 1 M sodium hydroxide solution, then extracted with 3 mL of ethyl acetate three times. The combined organic layers were washed with 3 mL of brine, dried over anhydrous sodium sulfate, filtered, and concentrated under reduced pressure to dryness to obtain the crude product. The crude product was purified by preparative thin-layer chromatography (eluent: petroleum ether: ethyl acetate = 1:1) to afford compound **138-6** as a yellow solid (20 mg, 0.05 mmol, yield = 47.92%). LC-MS (ESI): m/z 431.0/433.0 [M+H]⁺.

### Step 5

To a mixed solution of compound **138-6** (20 mg, 0.05 mmol), 2-[(1E)-2-ethoxyvinyl]-4,4,5,5-tetramethyl-1,3,2-dioxaborolane (13.68 µL, 0.06 mmol), tricyclohexylphosphine (1.30 mg, 0.001 mmol), potassium carbonate (19.23 mg, 0.14 mmol), 1,2-dioxane (1 mL), and water (0.2 mL), tris[(1E,4E)-1,5-diphenylpenta-1,4-diene-3-one]dipalladium(0) (4.25 mg, 0.001 mmol) were added. The mixture was degassed and purged with nitrogen three times, then stirred under nitrogen atmosphere at 90°C for 4 hours. Then, hydrochloric acid (185.50 µL, 6 M, 0.37 mmol) was added and the mixture was stirred at 70°C for 2 hours. The reaction mixture was quenched with 2 mL of saturated sodium bicarbonate solution and extracted with 4 mL of ethyl acetate three times. The combined organic layers were washed with 3 mL of brine, dried over anhydrous sodium sulfate, filtered, and concentrated under reduced pressure to dryness to obtain the crude product. The crude product was purified by preparative HPLC (Phenomenex Gemini column 150 mm × 25 mm × 10 µm, eluent: 30% to 60% (v/v) acetonitrile in water containing 0.025% ammonium bicarbonate) to afford compound **138** as a white solid (1 mg, 0.002 mmol, yield = 4.9%). LC-MS (ESI): m/z: 377.1 [M+H] ⁺. ¹H NMR (400 MHz, DMSO-*d*₆) δ: 13.32 (s, 1H), 8.30 (d, *J =* 7.2 Hz, 1H), 7.59-7.52 (m, 2H), 6.62 (s, 2H), 5.68-5.63 (m, 1H), 5.25 (d, *J =* 10.6 Hz, 1H), 2.09 (s, 3H), 1.98 (s, 3H), 1.97-1.90 (m, 3H).

### Example 5

### Synthesis of compound 2i

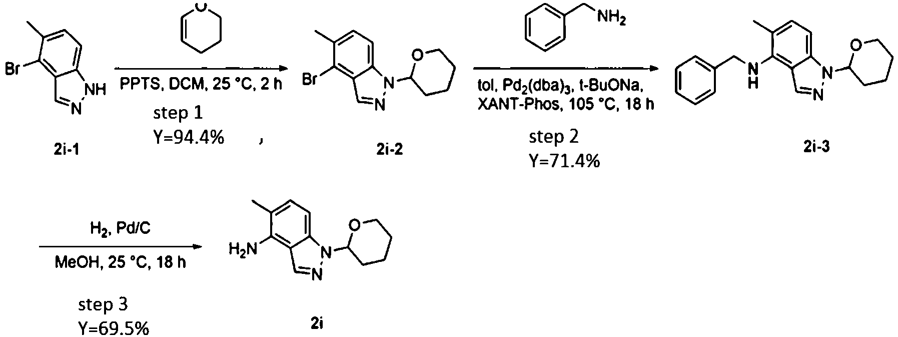

### Step 1

To a solution of compound **2i-1** (2.5 g, 11.84 mmol) and 3,4-dihydro-2H-pyran (2.17 mL, 23.69 mmol) in CH₂Cl₂ (25 mL), *p*-toluenesulfonic acid monohydrate (0.23 g, 1.18 mmol) was added. The resulting mixture was stirred at 25 °C for 2 hours. LCMS analysis showed complete consumption of the starting material and formation of the desired product. The mixture was concentrated under reduced pressure to dryness to obtain the crude product. The crude product was purified by silica gel chromatography (eluent: petroleum ether:EtOAc = 0 to 5%) to afford compound **2i-2** as a white solid (3.3 g, 11.18 mmol, yield = 94.4%).

### Step 2

To a mixture of compound **2i-2** (900 mg, 3.05 mmol), benzylamine (0.35 mL, 3.20 mmol), sodium tert-butoxide (879.02 mg, 9.15 mmol), and Xantphos (352.85 mg, 0.61 mmol) in toluene (10 mL), Pd₂(dba)₃ (279.2 mg, 0.30 mmol) was added. The mixture was degassed and purged with N₂ three times, then stirred at 105°C under N₂ atmosphere for 18 hours. LCMS analysis showed complete consumption of the starting material and formation of the desired product. The reaction mixture was poured into water (10 mL) and extracted with EtOAc (30 mL) three times. The combined organic layers were washed with brine (20 mL), dried over anhydrous Na₂SO₄, filtered, and concentrated under reduced pressure to dryness to obtain the crude product. The crude product was purified by silica gel chromatography (eluent: petroleum ether:EtOAc = 0 to 15%) to afford compound **2i-3** as a yellow oil (0.7 g, 2.18 mmol, yield = 71.4%).

### Step 3

To a mixture of compound **2i-3** (700 mg, 2.18 mmol) in MeOH (8 mL), Pd(0) (200 mg, 0.19 mmol) was added. The mixture was degassed and purged with H₂ three times, then stirred under H2 atmosphere at 25°C for 18 hours. LCMS analysis showed complete consumption of the starting material and formation of the desired product. The reaction mixture was filtered through a diatomaceous earth pad, and the pad was washed with MeOH (10 mL) twice. The filtrate was concentrated under reduced pressure to dryness to obtain the crude product. The crude product was purified by silica gel chromatography (eluent: petroleum ether:EtOAc = 0 to 20%) to afford compound **2i** as a white solid (350 mg, 1.51 mmol, yield = 69.5%). LC-MS (ESI): m/z = 232.2 [M+H]⁺.

### Example 6

Other compounds were prepared following procedures similar to those in Examples 1 to 5 above.

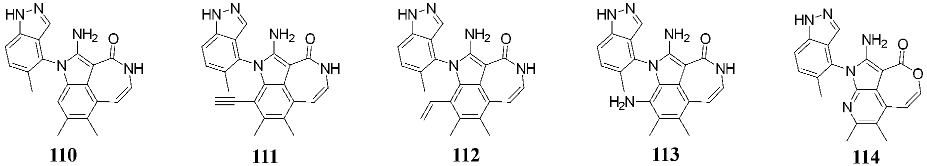

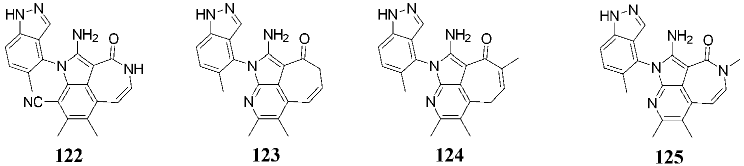

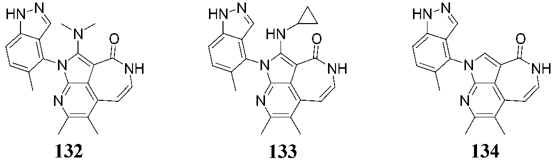

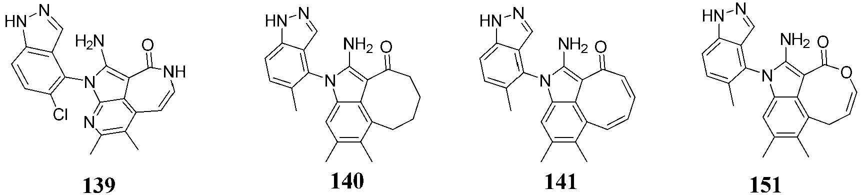

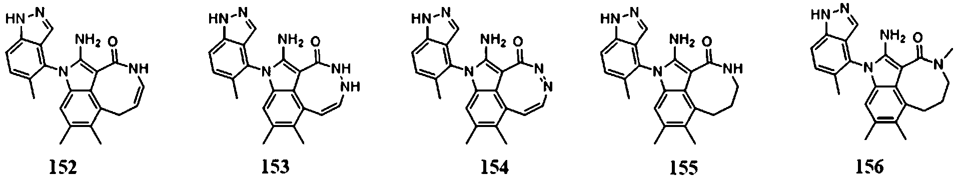

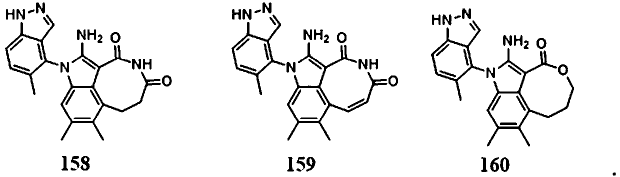

### Biological Examples

### Example X: ADP-Glo Enzyme Assay

The activity of Mytl kinase was measured by detecting ATP hydrolysis using recombinant human Mytl kinase and the commercially available ADP-Glo assay (Promega's ADP-Glo^{™} Kinase Assay, #G9101). Briefly, 5 µL of recombinant human Mytl (full-length PKMYT1 recombinant human protein expressed in insect cells from Thermo Fisher (#A32902)) was prepared in a reaction buffer (70 mM HEPES, 3 mM MgCl2, 3 mM MnCl2, 50 pg/mL PEG 20000, 3 µM sodium orthovanadate, 1.2 mM DTT) and added to wells of a 384-well white polystyrene, flat-bottom, untreated microplate. Then, 5 µL of test compounds were added to the wells of the microplate, and the plate was briefly centrifuged and incubated at room temperature for 15 minutes. Ultrapure ATP solution (from Promega's ADP-Glo kit) was diluted in the reaction buffer, and 5 µL of the resulting dilution was added to the wells of the microplate, followed by a brief centrifugation and incubation at room temperature for 60 minutes. After 60 minutes, 5 µL of ADP-Glo reagent was added, and the microplate was briefly centrifuged, sealed, and incubated in the dark at room temperature for 40 minutes. Subsequently, 10 µL of kinase detection reagent was added to each well, and the microplate was briefly centrifuged, sealed, and incubated in the dark at room temperature for another 40 minutes. Luminescence was read using an Envision plate reader. IC₅₀ values and maximum percent inhibition for each test inhibitor compound were calculated.

### Example Y: NanoBRET Assay

HEK293 cell lines transiently expressing the NanoLuc-PKMYT1 fusion construct were seeded into wells of a 3 84-well plate. Cells were pretreated with NanoBRET tracer, then treated with test compounds for 2 hours. BRET signals were measured on an Envision multimode plate reader at 450 nm and 610 nm. IC₅₀ values were calculated, and IC₅₀ curves were plotted using GraphPad Prism with a four-parameter nonlinear regression model.

### Example Z: Cell Proliferation Assay

The sensitivity of cancer cell lines with normal or elevated CCNE1 levels to test compounds was evaluated in a cell proliferation assay. Cell lines were maintained in ATCC-recommended culture media supplemented with 10% FBS and 1% penicillin/streptomycin in a humidified 5% CO₂ incubator. All test compounds were stored as 20 mM DMSO stock solutions.

On Day 0, CCNE1-wild type (wt) and CCNE1-amplified (amp) cells were seeded into 384-well plates and incubated for 16 to 24 hours in a humidified 5% CO₂ incubator. On Day 1, test compounds were dispensed into wells at specified concentrations (n=2), with DMSO volume normalized to the highest concentration. Plates with compound-treated cells were incubated for 7 days in a humidified 5% CO₂ incubator.

On Day 7, plates were removed from the incubator and equilibrated to room temperature. Relative inhibition was assessed by adding CellTiter-Glo reagent. Data were plotted as a percentage of the DMSO control for each compound in each cell line. IC₅₀ values were calculated using a four-parameter nonlinear regression model (GraphPad Prism).

**Table 1: Cell viability data**

| Compound no. | NanoBRET IC₅₀ (nM) | HCC1569 IC₅₀ (nM) |
|---|---|---|
| **126** | 5.50 | 666.00 |
| **136** | 21.59 | >1000 |
| **137** | 22.54 | 565.6 |
| **138** | 19.80 | >1000 |
| **Rp6306** | 1.6 | 18 |

According to the data results shown in Table 1, the compounds of the present disclosure demonstrated the ability to effectively bind to Myt1 intracellularly in the NanoBRET assay, and exhibited activity in inhibiting cell proliferation in the CCNE1-amplified breast cancer cell line HCC1569.

Although the present invention has been described and illustrated with reference to specific embodiments of the present invention, these descriptions and illustrations are not restrictive. It should be understood by those skilled in the art that various changes may be made and equivalents may be substituted without departing from the true spirit and scope of the present invention as defined by the appended claims. The illustrations may not necessarily be drawn to scale. Due to manufacturing processes and tolerances, there may be differences between the process reproduction in the present invention and the actual equipment. There may be other embodiments of the present invention that are not specifically described. The specification and drawings should be regarded as illustrative rather than restrictive. Modifications may be made to make specific situations, materials, material compositions, methods or processes suitable for the goals, spirit and scope of the present invention. All such modifications are intended to be within the scope of the appended claims. Although the methods disclosed herein have been described with reference to specific operations performed in a specific order, it should be understood that these operations may be combined, subdivided or reordered to form equivalent methods without departing from the teachings of the present invention. Accordingly, unless specifically indicated herein, the order and grouping of operations are not limitations of the present invention.

## Claims

1. A compound of formula (I), 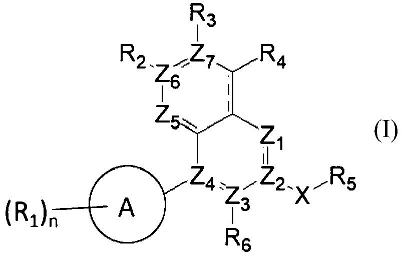 wherein:
ring A is indazolyl;
R₁ is hydrogen, halogen, hydroxyl, oxo, cyano, nitro, amino, alkyl, alkoxy, cycloalkyl, haloalkyl, haloalkoxy, halocycloalkyl, -B(OH)₂, -S(=O)-alkyl, -S(=O)₂-alkyl, -S(=O)NH₂, - S(=O)₂NH₂, -P(=O)-dialkyl, -C(=O)NH₂, -C(=O)alkyl, -C(=O)alkoxy, boronic acid group, heterocyclyl, aryl, or heteroaryl, wherein the alkyl, alkoxy, cycloalkyl, haloalkyl, haloalkoxy, halocycloalkyl, heterocyclyl, aryl, or heteroaryl groups are optionally substituted with substituents independently selected from halogen, hydroxyl, amino, alkyl, aryl, heteroaryl, or heterocyclyl;
Z₁ is a direct bond;
Z₂, Z₃, and Z₄ are independently CRₐ or N;
Z₅ is -C(=O)-, N, or CRₐ;
Z₆ and Z₇ are independently CRₐ or N;
wherein the position of the double bonds on the bicyclic ring connected to ring A varies depending on whether Z₂ to Z₇ are N or -C(=O)-; and, wherein Rₐ is hydrogen, alkyl, hydroxyalkyl, alkenyl, alkynyl, amino, or cyano;
X is -C(=O)- or NR_{c}, wherein R_{c} is heterocyclyl, aryl, or heteroaryl, and wherein the heterocyclyl, aryl, or heteroaryl groups are optionally substituted with substituents independently selected from hydroxyl, amino, alkyl, aryl, heteroaryl, or heterocyclyl;
R₂, R₃, and R₄ are independently hydrogen, alkyl, -C(=O)NH₂, amino, alkylamino, or dialkylamino, wherein the alkyl, amino, alkylamino, or dialkylamino groups are optionally substituted with substituents independently selected from hydroxyl, amino, alkyl, aryl, heteroaryl, or heterocyclyl;
R₅ is alkyl, amino, alkylamino, or dialkylamino, wherein the alkyl, amino, alkylamino, or dialkylamino groups are optionally substituted with substituents independently selected from hydroxyl, amino, alkyl, aryl, heteroaryl, or heterocyclyl; and R₅ together with R₄ forms a 5- to 8-membered heteroaryl or heterocyclic ring, wherein the heteroaryl or heterocyclic ring is optionally substituted with substituents independently selected from halogen, cyano, alkoxy, carbonyl, hydroxyl, amino, alkyl, aryl, heteroaryl, heterocyclyl, or cycloalkyl groups;
R₆ is hydrogen, amino, alkylamino, or dialkylamino; or, R₆ together with the attached Z₃ forms a -C(=O)- group; or, R₆ together with X forms a 5- to 8-membered heteroaryl or heterocyclic ring, wherein the heteroaryl or heterocyclic ring is optionally substituted with substituents independently selected from halogen, cyano, alkoxy, carbonyl, hydroxyl, amino, alkyl, aryl, heteroaryl, heterocyclyl, or cycloalkyl groups;
n is 3 or 4;
or a pharmaceutically acceptable salt, geometric isomer, enantiomer, diastereomer, racemate, prodrug, solvate, or hydrate thereof,

2. The compound according to claim 1, having formula (IC), 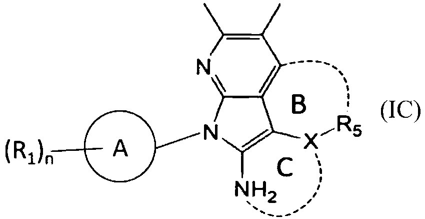 wherein:
ring A is indazolyl;
each R₁ is independently halogen, hydroxyl, oxo, cyano, nitro, amino, alkyl, alkoxy, cycloalkyl, haloalkyl, haloalkoxy, halocycloalkyl, -B(OH)₂, -S(=O)-alkyl, -S(=O)₂-alkyl, - S(=O)NH₂, -S(=O)₂NH₂, -P(=O)-dialkyl, -C(=O)NH₂, -C(=O)alkyl, -C(=O)alkoxy, boronic acid group, heterocyclyl, aryl, or heteroaryl, wherein the alkyl, alkoxy, cycloalkyl, haloalkyl, haloalkoxy, halocycloalkyl, heterocyclyl, aryl, or heteroaryl groups are optionally substituted with substituents independently selected from halogen, hydroxyl, amino, alkyl, aryl, heteroaryl, or heterocyclyl;
n is 3 or 4;
X is -C(=O)- or NR_{c}, wherein R_{c} is heterocyclyl, aryl, or heteroaryl;
R₅ is alkyl, amino, alkylamino, or dialkylamino, wherein the alkyl, amino, alkylamino, or dialkylamino groups are optionally substituted with substituents independently selected from hydroxyl, amino, alkyl, aryl, heteroaryl, or heterocyclyl;
ring B and Ring C do not exist simultaneously;
ring B is a 5- to 8-membered heteroaryl or heterocyclyl, wherein the heteroaryl and heterocyclyl are optionally substituted with substituents independently selected from halogen, cyano, alkoxy, carbonyl, hydroxyl, amino, alkyl, aryl, heteroaryl, heterocyclyl, or cycloalkyl; and
ring C is a 5- to 8-membered heteroaryl or heterocyclyl, wherein the heteroaryl and heterocyclyl are optionally substituted with substituents independently selected from halogen, cyano, alkoxy, carbonyl, hydroxyl, amino, alkyl, aryl, heteroaryl, heterocyclyl, or cycloalkyl.

3. The compound according to any one of claims 1-2, wherein X is -C(=O)-.

4. The compound according to any one of claims 1-2, wherein X is NR_{c}, and wherein R_{c} is heterocyclyl, aryl, or heteroaryl.

5. The compound according to any one of claims 1-4, wherein R₁ is selected from a group consisting of 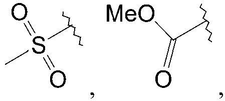
-CH₃, 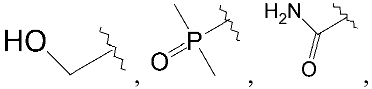
-CN,-OH, 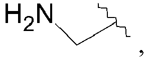
-OCH₃, -F, -Cl, -Br, 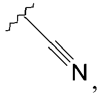
-CF₃, -CCl₃, -NO₂, 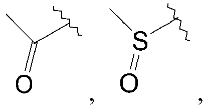 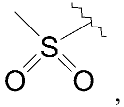
-B(OH)₂,

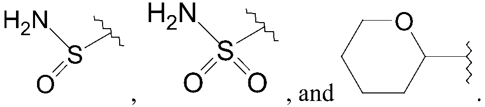

6. The compound according to claim 1, wherein Z₁ is a direct bond; Z₃ is C; at least one of Z₂ and Z₄ is N; Z₅ is -C(=O)-, N, or CRₐ, wherein Rₐ is hydrogen, alkyl, hydroxyalkyl, alkenyl, alkynyl, amino, or cyano; and Z₆ and Z₇ are independently C or N.

7. The compound according to claim 1, wherein R₂, R₃, and R₄ are independently hydrogen, alkyl, -C(=O)NH₂, or amino; R₅ is alkyl or amino; and R₆ is hydrogen or amino.

8. The compound according to claim 2, wherein R₅ is alkyl or amino.

9. The compound according to claim 2, wherein X is -C(=O)-; R₅ is alkyl or amino; ring B is a 6- or 7-membered heteroaryl or heterocyclyl, wherein the heteroaryl and heterocyclyl groups are optionally substituted with substituents independently selected from halogen, cyano, alkoxy, carbonyl, hydroxyl, amino, alkyl, aryl, heteroaryl, heterocyclyl, or cycloalkyl; and ring C does not exist.

10. The compound according to claim 1, selected from:

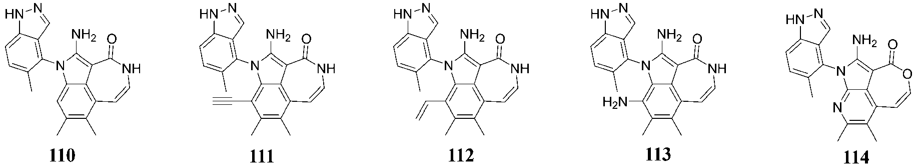

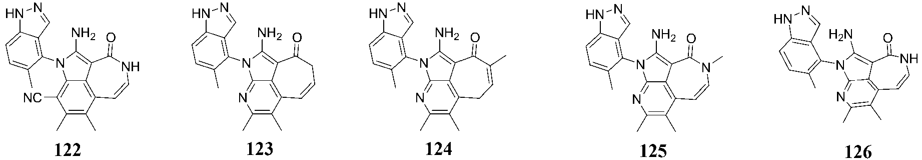

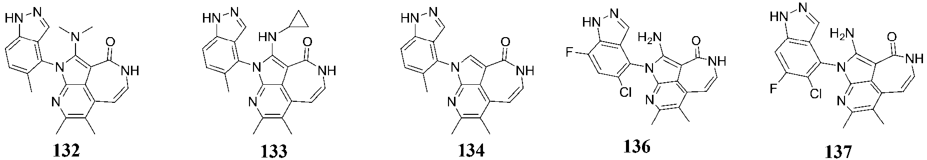

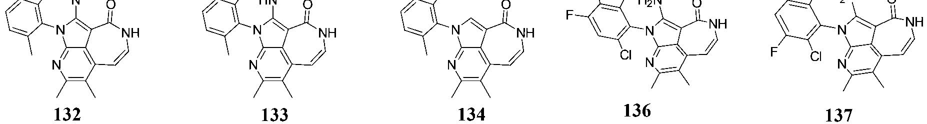

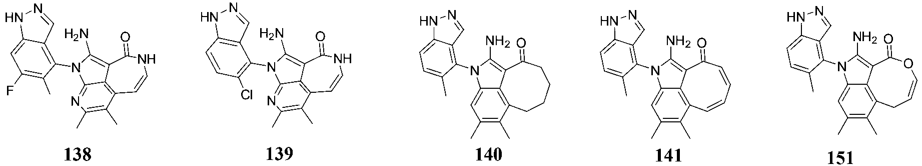

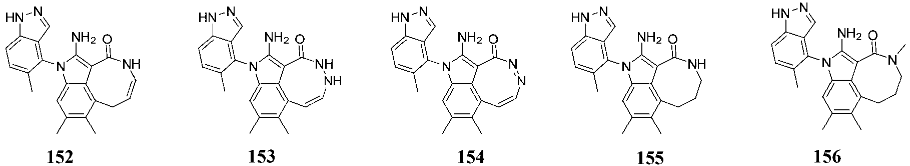

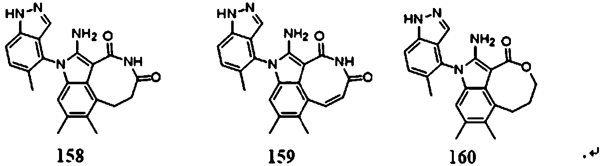

11. A pharmaceutical composition comprising a therapeutically effective amount of a compound or a pharmaceutically acceptable salt, geometric isomer, enantiomer, diastereomer, racemate, prodrug, solvate, or hydrate thereof according to any one of claims 1-10, and a pharmaceutically acceptable carrier or excipient.

12. Use of a compound or a pharmaceutically acceptable salt, geometric isomer, enantiomer, diastereomer, racemate, prodrug, solvate, or hydrate thereof according to any one of claims 1-11 in the manufacture of a medicament for treating a cancer in an individual in need thereof.

13. The use according to claim 12, wherein the compound or a pharmaceutically acceptable salt, geometric isomer, enantiomer, diastereomer, racemate, prodrug, solvate, or hydrate thereof is administered to the individual in combination with a second anticancer agent, surgical treatment, or an ionizing radiation therapy.

14. The use according to claim 12, wherein the second anticancer agent is selected from: targeted cancer drugs, such as trastuzumab, ramucirumab, bevacizumab, everolimus, tamoxifen, toremifene, fulvestrant, anastrozole, exemestane, lapatinib, letrozole, pertuzumab, ado-trastuzumab emtansine, palbociclib, cetuximab, panitumumab, ziv-aflibercept, regorafenib, lmatinib mesylate, lanreotide acetate, sunitinib, regorafenib, denosumab, alitretinoin, sorafenib, pazopanib, temsirolimus, everolimus, tretinoin, dasatinib, nilotinib, bosutinib, rituximab, alemtuzumab, ofatumumab, obinutuxumab, ibrutinib, idelalisib, blinatumomab, soragenib, crizotinib, erlotinib, gefitinib, afatinib dimaleate, ceritnib, ramucirumab, nivolumab, pembrolizumab, osimertinib, and necitumumab; alkylating agents, such as busulfan, chlorambucil, cyclophosphamide, iphosphamide, melphalan, nitrogen mustard, streptozocin, thiotepa, uracil nitrogen mustard, triethylenemelamine, temozolomide, and 2-chloroethyl-3-sarcosinamide-1-nitrosourea (SarCNU); antibiotics or plant alkaloids, such as actinomycin-D, bleomycin, cryptophycin, daunorubicin, doxorubicin, idarubicin, irinotecan, L-asparaginase, mitomycin-C, mitramycin, navelbine, paclitaxel, docetaxel, topotecan, vinblastine, vincristine, teniposide (VM-26), and etoposide (VP-16); hormones or steroids, such as 5α-reductase inhibitor, aminoglutethimide, anastrozole, bicalutamide, chlorotrianisene, diethylstilbestrol (DES), dromostanolone, estramustine, ethinyl estradiol, flutamide, fluoxymesterone, goserelin, hydroxyprogesterone, letrozole, leuprolide, medroxyprogesterone acetate, megestrol acetate, methyl prednisolone, methyltestosterone, mitotane, nilutamide, prednisolone, arzoxifene (SERM-3), tamoxifen, testolactone, testosterone, triamicnolone, and zoladex; synthetics, such as all-trans retinoic acid, carmustine (BCNU), carboplatin (CBDCA), lomustine (CCNU), cis-diaminedichloroplatinum (cisplatin), dacarbazine, gliadel, hexamethylmelamine, hydroxyurea, levamisole, mitoxantrone, o,ρ'-dichlorodiphenyldichloroethane (o,ρ'-DDD) (also known as lysodren or mitotane), oxaliplatin, porfimer sodium, procarbazine, and imatinib mesylate (Gleevec^{®}); antimetabolites, such as chlorodeoxyadenosine, cytosine arabinoside, 2'-deoxycoformycin, fludarabine phosphate, 5-fluorouracil (5-FU), 5-fluoro-2'-deoxyuridine (5-FUdR), gemcitabine, camptothecin, 6-mercaptopurine, methotrexate, and thioguanine; and biologics, such as alpha interferon, BCG (Bacillus Calmette-Guerin), granulocyte colony stimulating factor (G-CSF), granulocyte-macrophage colony-stimulating factor (GM-CSF), interleukin-2, and herceptin.

15. The use according to claim 12, wherein the cancer includes cardiac cancers, such as sarcoma (e.g., angiosarcoma, fibrosarcoma, rhabdomyosarcoma, and liposarcoma), myxoma, rhabdomyoma, fibroma, lipoma and teratoma; lung cancers, such as bronchogenic carcinoma (e.g., squamous cell carcinoma, undifferentiated small cell carcinoma, undifferentiated large cell carcinoma, and adenocarcinoma), alveolar carcinoma (e.g., bronchiolar carcinoma), bronchial adenoma, sarcoma, lymphoma, chondromatous hanlartoma, and inesothelioma; gastrointestinal cancers, such as esophagus carcinoma (e.g., squamous cell carcinoma, adenocarcinoma, leiomyosarcoma, and lymphoma), stomach carcinoma (e.g., lymphoma, and leiomyosarcoma), pancreas carcinoma (e.g., ductal adenocarcinoma, insulinoma, glucagonoma, gastrinoma, carcinoid, and vipoma), small bowel carcinoma (e.g., adenocarcinoma, lymphoma, carcinoid, Karposi's sarcoma, leiomyoma, hemangioma, lipoma, neurofibroma, and fibroma), large bowel carcinoma (e.g., adenocarcinoma, tubular adenoma, villous adenoma, hamartoma, and leiomyoma); genitourinary tract cancers, such as kidney carcinoma (e.g., adenocarcinoma, Wilm's tumor [nephroblastoma], lymphoma, and leukemia), bladder and urethra carcinoma (e.g., squamous cell carcinoma, transitional cell carcinoma, and adenocarcinoma), prostate carcinoma (e.g., adenocarcinoma, and sarcoma), testis carcinoma (e.g., seminoma, teratoma, embryonal carcinoma, teratocarcinoma, choriocarcinoma, sarcoma, interstitial cell carcinoma, fibroma, fibroadenoma, adenomatoid tumors, and lipoma); liver cancers, such as hepatoma (e.g., hepatocellular carcinoma), cholangiocarcinoma, hepatoblastoma, angiosarcoma, hepatocellular adenoma, and hemangioma; bone cancers, such as osteogenic sarcoma (e.g., osteosarcoma), fibrosarcoma, malignant fibrous histiocytoma, chondrosarcoma, Ewing's sarcoma, malignant lymphoma (e.g., reticulum cell sarcoma), multiple myeloma, malignant giant cell tumor chordoma, osteochronfroma (e.g., osteocartilaginous exostoses), benign chondroma, chondroblastoma, chondromyxofibroma, osteoid osteoma, and giant cell tumors; nervous system cancers, such as skull carcinoma (e.g., osteoma, hemangioma, granuloma, xanthoma, and osteitis deformans), meninx carcinoma (e.g., meningioma, meningiosarcoma, and gliomatosis), brain carcinoma (e.g., astrocytoma, medulloblastoma, glioma, ependymoma, germinoma (pinealoma), glioblastoma multiform, oligodendroglioma, schwannoma, retinoblastoma, and congenital tumors), spinal cord neurofibroma, meningioma, glioma, and sarcoma; gynecological cancers, such as uterus carcinoma (e.g., endometrial carcinoma), cervix carcinoma (e.g., cervical carcinoma, and pre- tumor cervical dysplasia), ovary carcinoma (e.g., ovarian carcinoma [e.g., serous cystadenocarcinoma, mucinous cystadenocarcinoma, and unclassified carcinoma], granulosa-thecal cell tumors, Sertoli-Leydig cell tumors, dysgerminoma, and malignant teratoma), vulva carcinoma (e.g., squamous cell carcinoma, intraepithelial carcinoma, adenocarcinoma, fibrosarcoma, and melanoma), vagina carcinoma (e.g., clear cell carcinoma, squamous cell carcinoma, botryoid sarcoma (e.g., embryonal rhabdomyosarcoma], and fallopian tube carcinoma); hematologic cancers, such as blood carcinoma (myeloid leukemia [acute and chronic], acute lymphoblastic leukemia, chronic lymphocytic leukemia, myeloproliferative diseases, multiple myeloma, and myelodysplastic syndrome), Hodgkin's disease, non-Hodgkin's lymphoma [malignant lymphoma]; skin cancers, such as malignant melanoma, basal cell carcinoma, squamous cell carcinoma, Karposi's sarcoma, moles dysplastic nevi, lipoma, angioma, dermatofibroma, keloids, psoriasis; adrenal gland cancers, such as neuroblastoma; or breast cancer.

16. A process for preparing a compound of formula (I) or a pharmaceutically acceptable salt, geometric isomer, enantiomer, diastereomer, racemate, prodrug, solvate, or hydrate thereof according to any one of claims 1-10, the process comprising:
a. reacting a compound of formula (II) 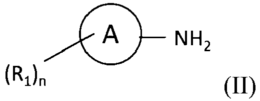 with a pyridine of formula (III) 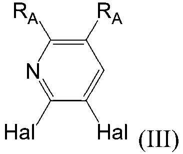 in the presence of a catalyst to obtain a compound of formula (IV) 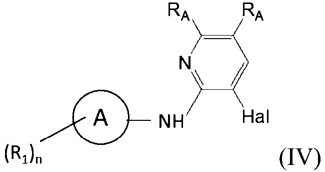 wherein ring A, R₁, and n are as defined in claim 1, Hal is halogen, and R_{A} is alkyl;
b. reacting the compound of formula (IV) with a coupling agent containing cyano to obtain a compound of formula (I'), 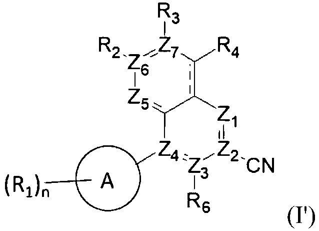
wherein ring A, Z₁, Z₂, Z₃, Z₄, Z₅, Z₆, Z₇, R₁, R₂, R₃, R₄, R₆, and n are as defined in claim 1; and
allowing the compound of formula (I') to undergo an acylation reaction in the presence of an acid to obtain the compound of formula (I).
